# EUROPEAN PATENT APPLICATION

(11) **EP 4 534 542 A1**
(43) Date of publication of application: **09.04.2025**
(21) Application number: 23383012.4
(22) Date of filing: 02.10.2023
(51) Int. Cl.: C07H 15/203, A61P 35/00, A61K 31/7028

(54) **THIOSUGAR BASED ISOTHIOCYANATES AND USES THEREOF**

(71) Applicant: Universidad de Sevilla, 41013 Sevilla (ES); Consejo Superior De Investigaciones Científicas, 28006 Madrid (ES); Servicio Andaluz de Salud, 41071 Sevilla (ES)
(72) Inventor: FERNÁNDEZ FERNÁNDEZ, Inmaculada, 41013 Sevilla (ES); KHIAR EL WAHABI, Noureddine, 28006 Madrid (ES); PEREZ SIMON, Jose Antonio, 41013 Sevilla (ES); VALDIVIA GIMÉNEZ, Victoria Esther, 41013 Sevilla (ES); RECIO JIMÉNEZ, Rocío, 41013 Sevilla (ES); PRIETO RAMÍREZ, Luis Alberto, 41013 Sevilla (ES); RODRIGUEZ GIL, Alfonso, 41013 Sevilla (ES); LOPEZ LAZARO, Miguel, 41013 Sevilla (ES); CALDERÓN MONTAÑO, José Manuel, 41013 Sevilla (ES); LEÓN MARTÍNEZ, Rafael, 28006 Madrid (ES); CALDERON RUIZ, Rocío, 41071 Sevilla (ES); BEJARANO GARCÍA, José Antonio, 41071 Sevilla (ES)
(74) Representative: Hoffmann Eitle

(57) **Abstract**

The present disclosure relates to novel small molecules, particularly, thiosugar based isothiocyanate derivatives of formula I: 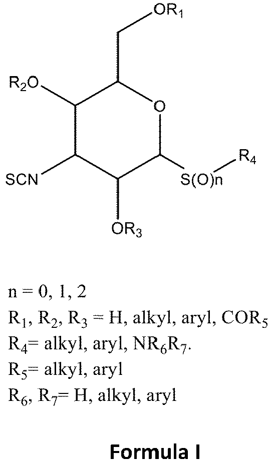 and their use thereof in a method of treatment by therapy, preferably, in a method of treating cancer.

## Description

### Field of the invention

The present disclosure relates to novel small molecules, particularly, isothiocyanate derivatives of formula **I** and their use as lead compounds for manufacturing a medicament or a pharmaceutical composition for therapy, in particular, for treating cancer.

### Background of the invention

For decades, various plants have been studied as sources of biologically active compounds. Compounds with anticancer and antimicrobial properties are the most frequently desired. Cruciferous plants, including Brussels sprouts, broccoli, and wasabi, have a special role in the research studies.

Studies have shown that consumption of these plants reduce the risk of lung, breast, and prostate cancers. The high chemopreventive and anticancer potential of cruciferous plants results from the presence of a large amount of glucosinolates, which, under the influence of the enzyme myrosinase, undergo a Lossen-type rearrangement to biologically active isothiocyanates (ITCs). Natural isothiocyanates, such as benzyl isothiocyanate, phenethyl isothiocyanate, or the best-tested sulforaphane, possess anticancer activity at all stages of the carcinogenesis process, and show antibacterial activity. Methods of synthesizing sulforaphane, alongside its natural or synthetic bifunctional analogues featuring sulfinyl, sulfanyl, sulfonyl, phosphonate, phosphinate, phosphine oxide, carbonyl, ester, carboxamide, ether, or additional isothiocyanate functional groups, and with the unbranched alkyl chain containing 2-6 carbon atoms, have been discussed in reviews such as Janczewski's work Sulforaphane and Its Bifunctional Analogs: Synthesis and Biological Activity. Molecules 2022, 27, 1750. https://doi.org/10.3390/ molecules27051750. The biological activity of these compounds is also reported in such review.

The present invention provides a novel class of isothiocyanate derivatives of formula I and their use as lead compounds for manufacturing a medicament or a pharmaceutical composition for therapy, in particular, for treating cancer.

### BRIEF DESCRIPTION OF THE DRAWINGS

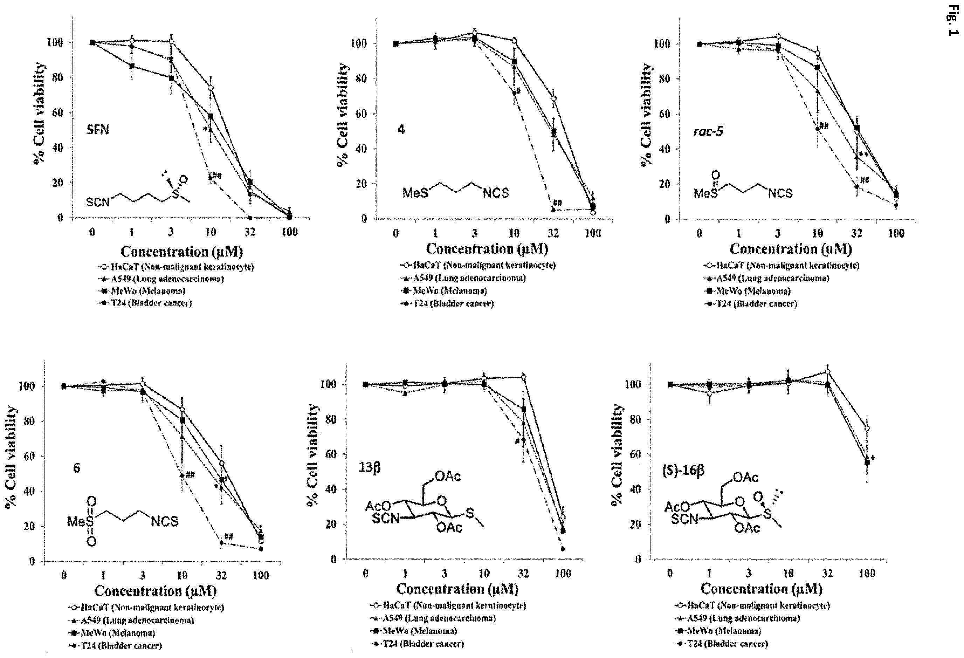

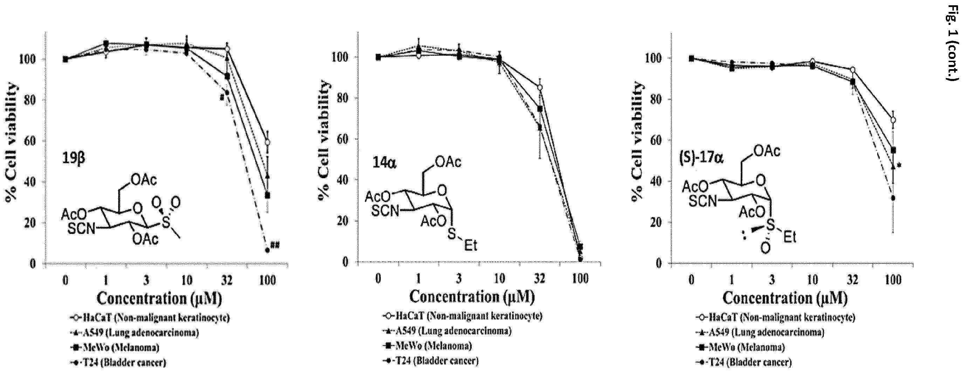

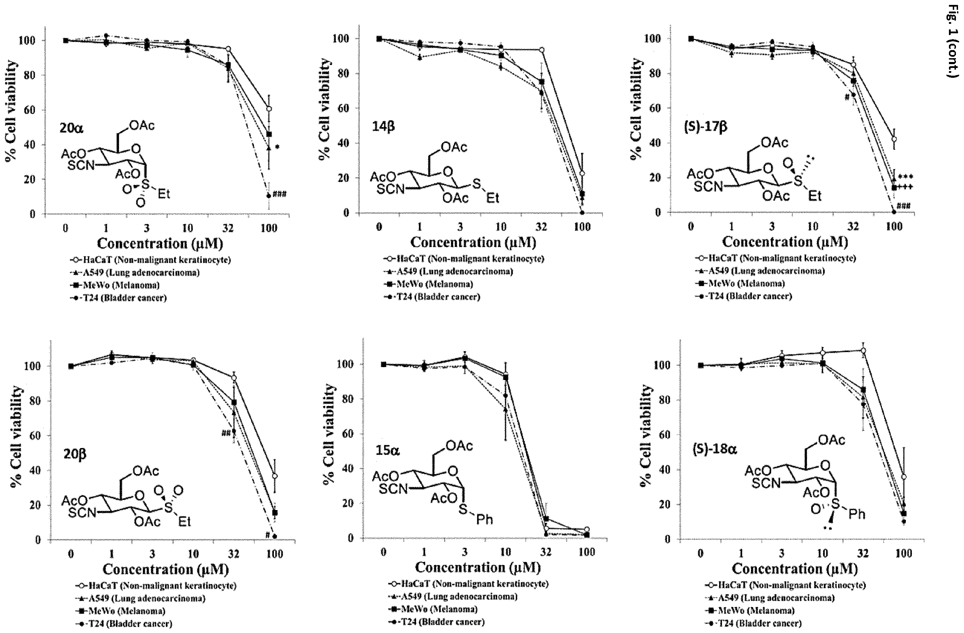

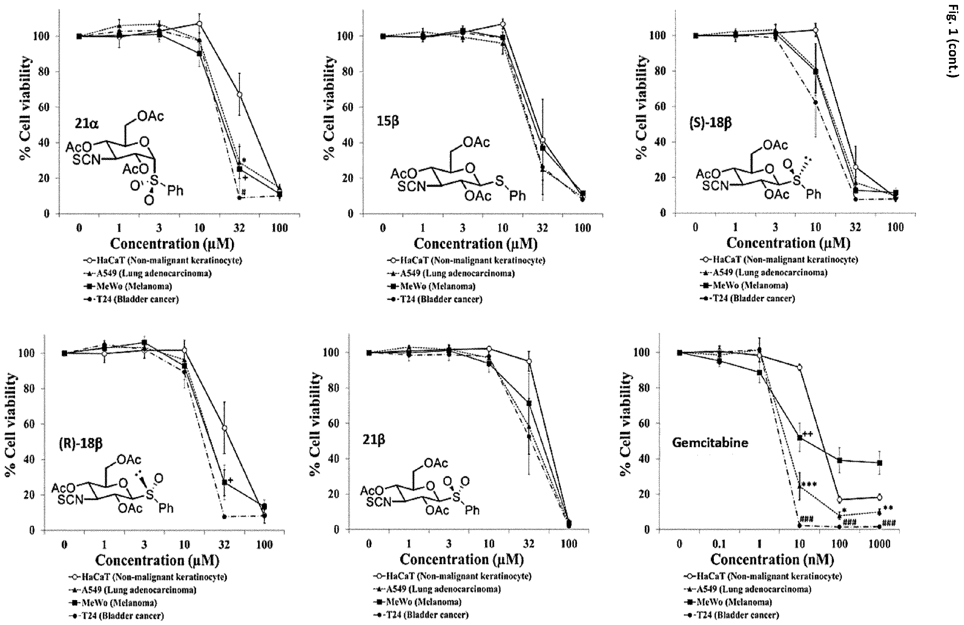

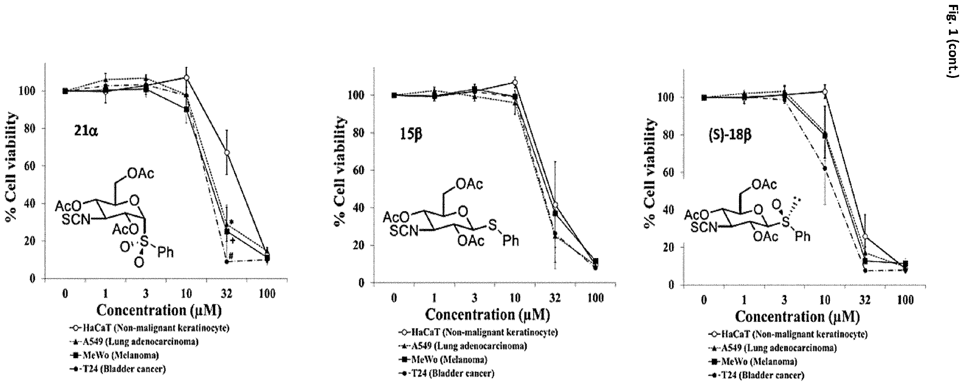

**Fig. 1****.** Evaluation of cytotoxic activity of natural isothiocyanates (SFN, **4, rac-5, 6)**, thiosugar based isothiocyanates and gemcitabine on cancer line cells [A549 (lung adenocarcinima), MeWo (melanoma) and T24 (bladder cancer)] and healthy cells [HaCaT (non-malignant keratinocyte)]. Cells were exposed to several concentrations of compounds for 72 h. Cell viability was determined with the resazurin assay. Results are representative of at least three independent experiments. Values are expressed in mean with SEM and p values (paired t-test) indicated.

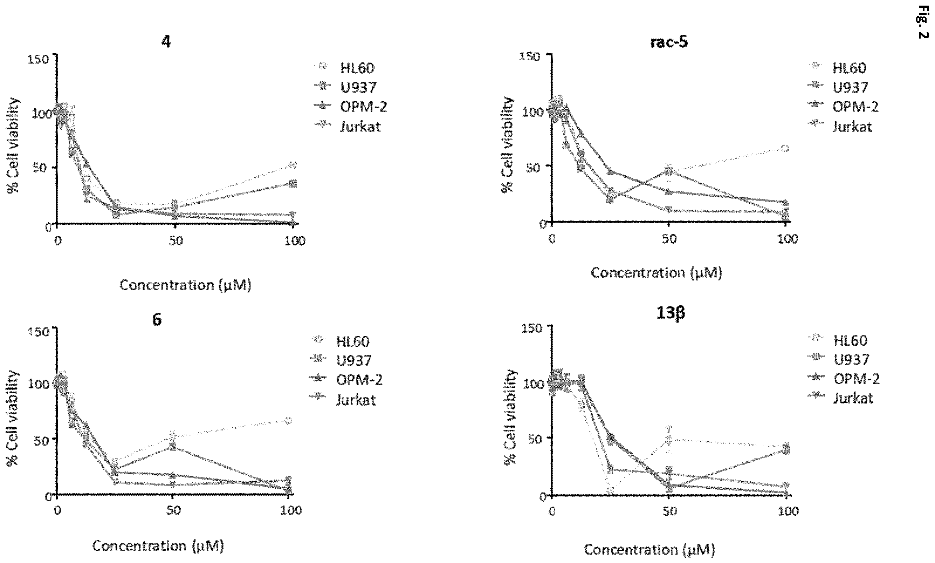

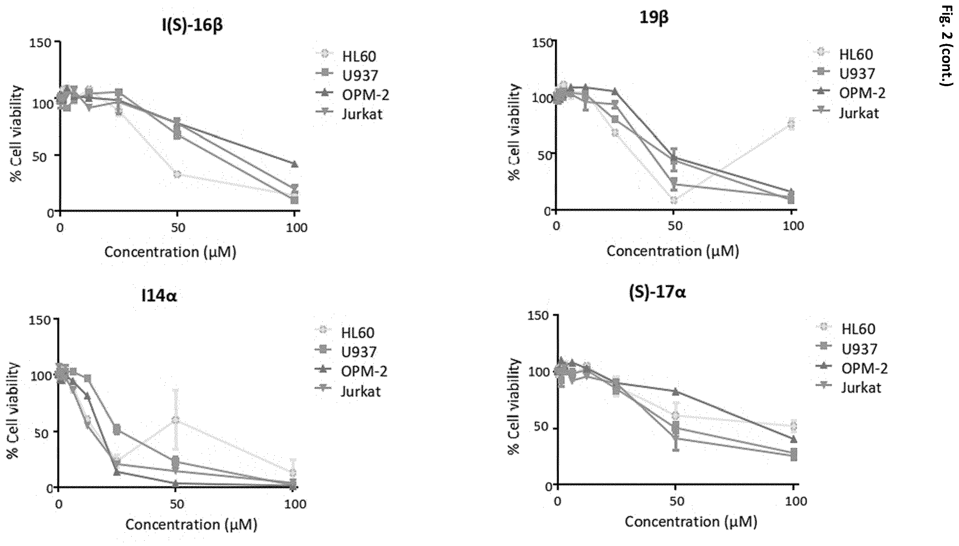

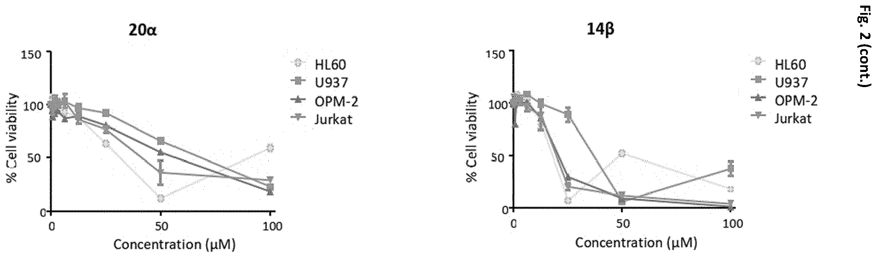

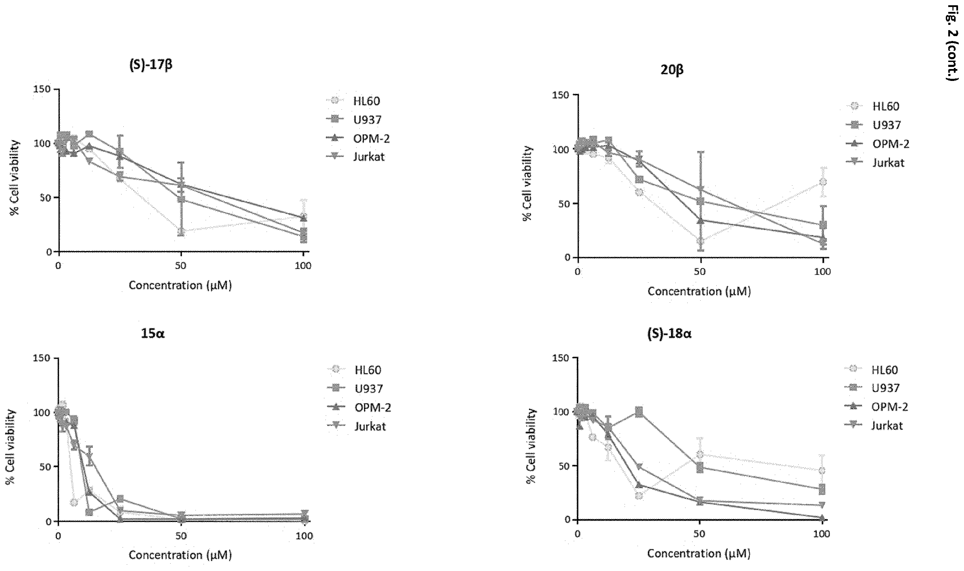

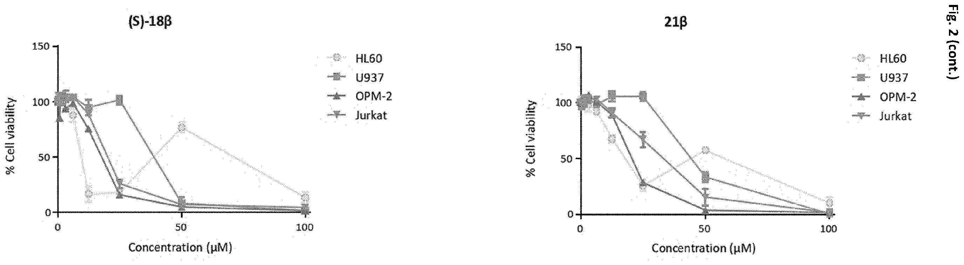

**Fig. 2****.** Evaluation of antileukemic activity of natural isothiocyanates (**4, rac-5, 6)** and thiosugar based isothiocyanates **(13β**, **(Ss)-16β, 19β, 14α, (*S*ₛ)-17α, 20α, 14β, (*S*ₛ)-17β, 20β, 15α, (*S*ₛ)-18α, (Ss)-18P, 21β)** on acute myeloid leukaemia (AML), multiple myeloma (MM), and acute lymphoblastic leukaemia (ALL) cell lines. Cell viability analysis determined by **MTT assay** in HL60 (AML), U937 (AML), OPM-2 (MM) and Jurkat (ALL) after incubation with them (0 - 100 µM) for 18 h.

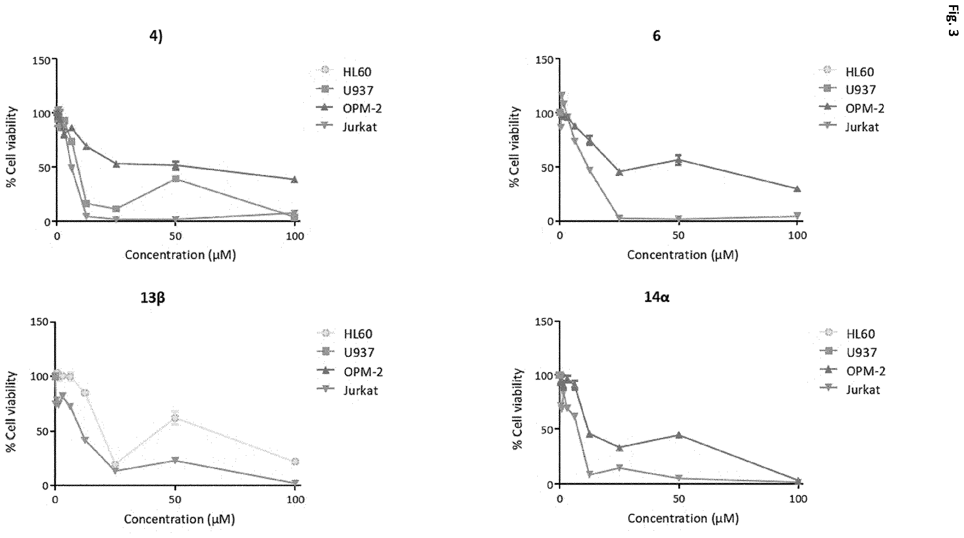

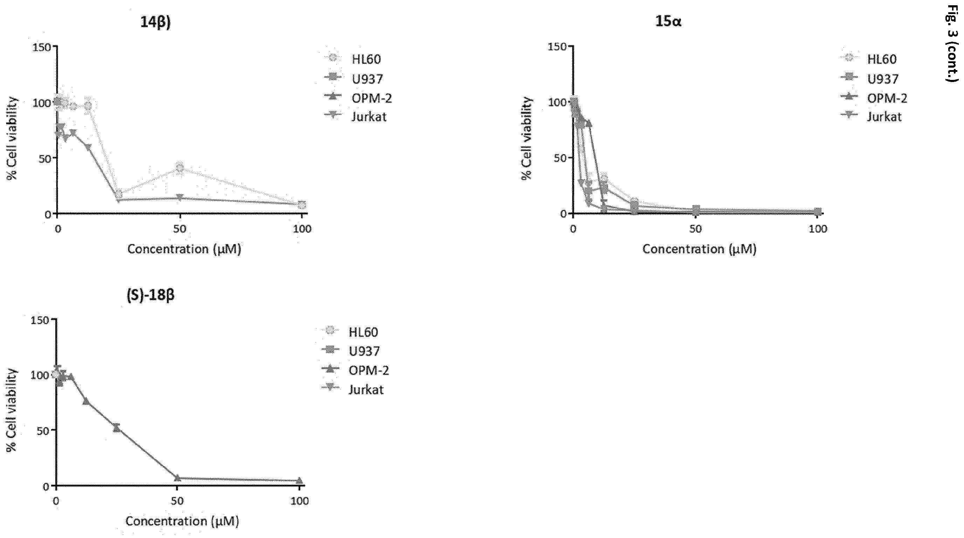

**Fig. 3****.** Evaluation of antileukemic activity of natural isothiocyanates (**4** and **6)** and thiosugar based isothiocyanates (**13β**, **14α, 14β, 15α, (Sₛ)-18β)** on acute myeloid leukaemia (AML), multiple myeloma (MM), and acute lymphoblastic leukaemia (ALL) cell lines. Cell viability analysis determined by **flow cytometry** in HL60 (AML), U937 (AML), OPM-2 (MM) and Jurkat (ALL) after incubation with them (0-100 µM) for 18 h.

### Description of the invention

The present disclosure is based, at least in part, in the unexpected discovery that isothiocyanate derivatives of formula I below: 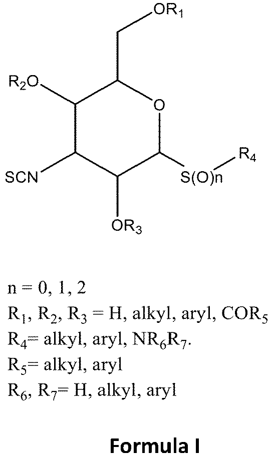 have activation capacity of the NrF2 factor (as shown in Table 1), cytotoxic activity against solid tumours (as shown in Table 2) and/or antileukemic activity (as shown in Table 3). The results of this invention indicate that these compounds are potential lead compounds for use as therapeutic agents for treating cancers.

Accordingly, it is the first aspect of this invention to provide a compound of formula I (from hereinafter refer to as "compound of the invention"):

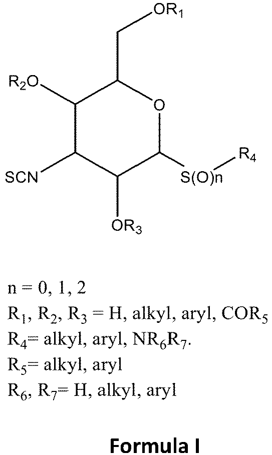

In a preferred embodiment, the compound of the invention is characterized by:
a) R₁ to R₃ being independently selected from a C₁-C₄ alkyl (preferably a C₁-C₂ alkyl), an aryl such as a phenyl group, or an acetyl group (-COCH₃); and
b) R₄ being selected from a C₁-C₄ alkyl (preferably a C₁-C₂ alkyl), or an aryl such as a substituted or unsubstituted phenyl group.

In another preferred embodiment, the compound of the invention is characterized by:
c) R₁ to R₃ being an acetyl group (-COCH₃); and
d) R4 being selected from a C₁-C₄ alkyl (preferably a C₁-C₂ alkyl), or an aryl such as a substituted or unsubstituted phenyl group.

In another preferred embodiment, the compound of the invention is characterized by having the following chemical structure: 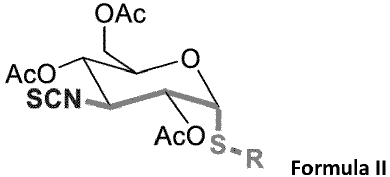 wherein Ac shall be understood as an acetyl group (-COCH₃), and R can be selected from a methyl group (compound **13α**), an ethyl group (compound **14α**), or a phenyl group (compound **15α**).

In another preferred embodiment, the compound of the invention is characterized by having the following chemical structure: 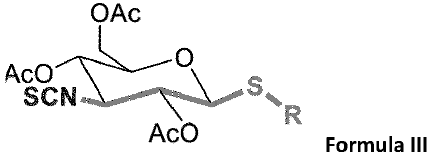 wherein Ac shall be understood as an acetyl group (-COCH₃), and R can be selected from a methyl group (compound **13β**), an ethyl group (compound **14β**), or a phenyl group (compound **15β**).

In another preferred embodiment, the compound of the invention is characterized by having the following chemical structure: 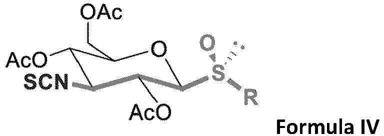 wherein Ac shall be understood as an acetyl group (-COCH₃), and R can be selected from a methyl group (compound *(S*ₛ)-**16β**, an ethyl group (compound (*S*ₛ)-**17β**), or a phenyl group (compound (*S*ₛ)-**18β**).

In another preferred embodiment, the compound of the invention is characterized by having the following chemical structure: 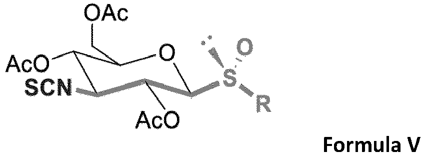 wherein Ac shall be understood as an acetyl group (-COCH₃), and R can be selected from an ethyl group (compound (*R*ₛ)-**17β)**, or a phenyl group (compound (*R*ₛ)-**18β**).

In another preferred embodiment, the compound of the invention is characterized by having the following chemical structure: 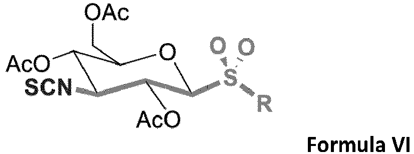 wherein Ac shall be understood as an acetyl group (-COCH₃), and R can be selected from a methyl group (compound **19β**), an ethyl group (compound 2**0β**), or a phenyl group (compound **21β**).

In another preferred embodiment, the compound of the invention is characterized by having the following chemical structure: 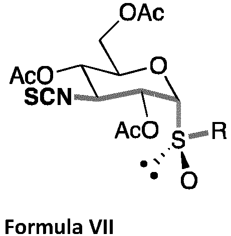 wherein Ac shall be understood as an acetyl group (-COCH₃), and R can be selected from an ethyl group (compound (*R*ₛ)-**17α**), or a phenyl group (compound (*R*ₛ)-**18α**).

In another preferred embodiment, the compound of the invention is characterized by having the following chemical structure: 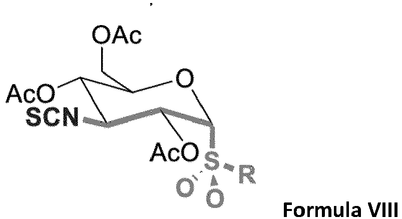 wherein Ac shall be understood as an acetyl group (-COCH₃), and R can be selected from an ethyl group (compound **20α**), or a phenyl group (compound **21α**).

Preferred compounds of the invention can be selected from any of the following list consisting of:

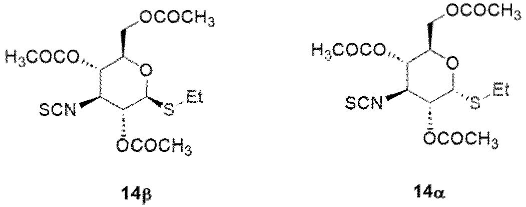

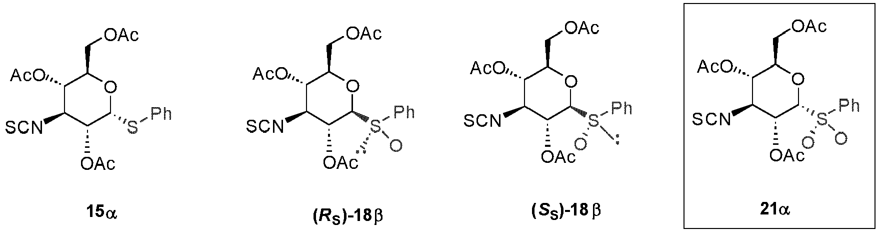

Any of the compounds of the invention can be in a free form or in the form of a pharmaceutically acceptable salt. Examples of pharmaceutically acceptable salts include inorganic acid salts such as hydrochloride, sulfate, nitrate, phosphate or hydrobromide, etc., organic acid salt such as acetate, fumarate, oxalate, citrate, methanesulfonate, benzenesulfonate, *p*-toluenesulfonate or maleate, etc. The compounds of the invention or their isomers, or pharmaceutically acceptable salts can be in any of its intramolecular salt or adduct, or its solvates or hydrates.

A second aspect of the invention refers to a composition, preferably a pharmaceutical composition, comprising a compound of the invention as defined in the first aspect of the invention.

Though not wishing to be bound by any particular theory, it is believed that the compounds of the present invention, as shown in tables 1 to 3, provide protection against oxidative and inflammatory stress, such as disturbances of systems that protect cells against oxidative damage, heat shock, and disturbances caused by protein misfolding. These protective mechanisms are thought to be at least partly mediated by the transcription factor Nrf2 which controls expression of genes of the human genome via the Keapl/Nrf2/ARE regulatory system. This system may be upregulated in many tissues by the compounds of the present invention. These compounds also have inhibitory effects on the growth of various types of cancer cells and can induce apoptosis of cancer cells.

Therefore, in a third aspect of the present invention, provides a method for treating an inflammatory disorder or a cancer in a subject, said method comprising administering to said subject an effective amount of a compound of the present invention. The method thus includes administering to the subject an effective amount of any of the compounds of the invention described above or a pharmaceutically acceptable salt thereof. The compounds of this disclosure are effective in treating cancer as shown in tables 2 and 3. Cancer that may be treated by the compounds of this disclosure includes solid tumours such as, but not limited to, lung cancer, in particular lung adenocarcinoma, melanoma, or bladder cancer, and haematological cancers such as, but not limited to, leukaemia, as acute myeloid leukaemia, multiple myeloma, acute lymphoblastic leukaemia and lymphoma. Other cancers or tumours treatable by the compounds of the present invention can be selected from the group consisting of brain tumours, nasopharyngeal carcinoma, breast cancer, colon cancer, liver cancer, stomach cancer, oesophageal cancer, and gastric cancer. The compounds of the present invention can also be used to treat autoimmune diseases. This is because the activation of Nrf2 is known to be useful as a beneficial method to treat autoimmune diseases based on the well-established anti-inflammatory role of Nrf2.

In some embodiments, the effective amount of the compounds of this invention administered to the subject is from about 1 to 100 mg/Kg body weight of the subject by oral ingestion, intravenous or intramuscular injection. The amount is administered to the subject at about 10, 20, 30, 40, 50, 60, 70, 80, 90 or 100 mg/Kg body weight of the subject per day, preferably about 30 to 70 mg/Kg body weight of the subject, such as 30, 40, 50, 60 or 70 mg/Kg body weight of the subject per day. The dose can be administered in a single dosage, or alternatively in more than one dosage.

In some embodiments, the method further includes the step of subjecting the cancer to a radiation treatment after administering the compounds of the invention.

In some embodiments, the method further includes the step of administering another agent that is known to improve the treatment of cancer, before, together with and/or after administering the compound of this invention. Examples of such agent include, but are not limited to, anti-cancer drug, anti-angiogenesis agent, anti-virus agent, antibiotic, analgesic, anti-anaemia drug, cytokine, granulocyte colony-stimulating factor (G-CSF), and anti-nausea drug and the like.

Examples of anti-cancer drug include, but are not limited to, paclitaxel, docetaxel, camptothecin (CPT), topotecan (TPT), irinotecan (CPT-11), Doxorubicin, daunorubicin, epirubicin, fluorouracil, cis-platin, cyclophosphamide, vinblastine, vincristine, ifosfamide, melphalan, mitomycin, methotrexate, mitoxantrone, teniposide, etoposide, bleomycin, leucovorin, cytarabine, dactinomycin, streptozocin, combretastatin A4-phosphate, SU5416, and the like. Examples of anti-angiogenesis agent include, but are not limited to, DS 4152, TNP-470, SU6668, endostatin, 2-methoxyestradiol, angiostatin, thalidomide, tetrathiomolybdate, linomide, IL-12, and the like. Examples of anti-virus agent include, but are not limited to, amantadine, rimantadine, and the like. Examples of analgesic include, but are not limited to, paracetamol such as *p*-acetylaminophenol, non-steroidal anti-inflammatory drug (NSAID) such as salicylates, and opioid drugs such as morphine and opium. Examples of anti-anemia drug includes, and is not limited to, erythropoietin.

As already indicated in the second aspect of the invention, this disclosure also provides a pharmaceutical composition for treating cancer; the composition comprises a therapeutically effective amount of a compound of this disclosure as shown above and optionally a pharmaceutically acceptable excipient.

Generally, the compound of this invention is present at a level of about 0.1% to 99% by weight, based on the total weight of the pharmaceutical composition. In some embodiments, the compound of this invention is present at a level of at least 1% by weight, based on the total weight of the pharmaceutical composition. In certain embodiments, the compound of this invention is present at a level of at least 5% by weight, based on the total weight of the pharmaceutical composition. In still other embodiments, the compound of this invention is present at a level of at least 10% by weight, based on the total weight of the pharmaceutical composition. In still yet other embodiments, the compound of this invention is present at a level of at least 25% by weight, based on the total weight of the pharmaceutical composition.

In some embodiments, the medicament of said pharmaceutical composition of this invention further includes an agent that is known to improve the treatment of cancer. Examples of such agent include, and are not limited to, anti-cancer drug, anti-angiogenesis agent, anti-virus agent, antibiotic, analgesic, anti-anomia drug, cytokine, granulocyte colony-stimulating factor, anti-nausea drug and the like.

The medicament or said pharmaceutical composition is prepared in accordance with acceptable pharmaceutical procedures, such as described in Remington's Pharmaceutical Sciences, 17th edition, ed. Alfonso R. Gennaro, Mack Publishing Company, Easton, Pa. (1985). Pharmaceutically acceptable excipients are those that are compatible with other ingredients in the formulation and biologically acceptable.

The compounds of this invention may be administered by any suitable route, for example, orally in capsules, suspensions or tablets or by parenteral administration. Parenteral administration can include, for example, systemic administration such as intramuscular, intravenous, subcutaneous, or intraperitoneal injection. The compound can also be administered transdermally either topically or by inhalation (e.g., intrabronchial, intranasal, oral inhalation or intranasal drops), or rectally, alone or in combination with conventional pharmaceutically acceptable excipients. In preferred embodiments, the compounds of this invention are administered orally (e.g., dietary) to the subject.

For oral administration, the compounds of the present invention may be formulated into tablets containing various excipients such as microcrystalline cellulose, sodium citrate, calcium carbonate, dicalcium phosphate, and glycine; along with various disintegrants such as starch, alginic acid and certain silicates; together with granulation binders like polyvinylpyrrolidone, sucrose, gelatine and acacia. Additionally, lubricating agents such as magnesium stearate, sodium lauryl sulfate and talc may be added. Solid composition may also be employed as fillers in gelatine capsules; preferred materials in this connection also include lactose or milk sugar as well as high molecular weight polyethylene glycols. When aqueous suspensions and/or elixirs are desired for oral administration, the active ingredient may be combined with various sweetening or flavouring agents, colouring matter or dyes, and if so desired, emulsifying and/or suspending agents as well, together with diluents such as water, ethanol, propylene glycol, glycerine and a combination thereof.

For parenteral administration, the compounds of the present invention may be formulated into liquid pharmaceutical compositions, which are sterile solutions, or suspensions that can be administered by, for example, intravenous, intramuscular, subcutaneous, or intraperitoneal injection. Suitable diluents or solvent for manufacturing sterile injectable solution or suspension include, but are not limited to, 1,3-butanediol, mannitol, water, Ringer's solution, and isotonic sodium chloride solution. Fatty acids, such as oleic acid and its glyceride derivatives are also useful for preparing injectables, as are natural pharmaceutically-acceptable oils, such as olive oil or castor oil. These oil solutions or suspensions may also contain alcohol diluent or carboxymethyl cellulose or similar dispersing agents. Other commonly used surfactants such as Tweens or Spans or other similar emulsifying agents or bioavailability enhancers that are commonly used in manufacturing pharmaceutically acceptable dosage forms can also be used for the purpose of formulation.

For topical administration, the medicament or said pharmaceutical compositions of this invention may be formulated into a variety of dosage forms for topical application. A wide variety of dermatologically acceptable inert excipients well known to the art may be employed. The topical compositions may include liquids, creams, lotions, ointments, gels, sprays, aerosols, skin patches, and the like. Typical inert excipients may be, for example, water, ethyl alcohol, polyvinyl pyrrolidone, propylene glycol, mineral oil, stearyl alcohol and gel-producing substances. All of the above dosages forms and excipients are well known to the pharmaceutical art. The choice of the dosage form is not critical to the efficacy of the composition described herein.

For transmucosal administration, the medicament or said pharmaceutical compositions of this invention may also be formulated in a variety of dosage forms for mucosal application, such as buccal and/or sublingual drug dosage units for drug delivery through oral mucosal membranes. A wide variety of biodegradable polymeric excipients may be used that are pharmaceutically acceptable, provide both a suitable degree of adhesion and the desired drug release profile, and are compatible with the active agents to be administered and any other components that may be present in the buccal and/or sublingual drug dosage units. Generally, the polymeric excipient comprises hydrophilic polymers that adhere to the wet surface of the oral mucosa. Examples of polymeric excipients include, but are not limited to, acrylic acid polymers and copolymers; hydrolyzed polyvinylalcohol; polyethylene oxides; polyacrylates; vinyl polymers and copolymers; polyvinylpyrrolidone; dextran; guar gum; pectins; starches; and cellulosic polymers.

Accordingly, this invention also provides methods of treating mammals, preferably humans, for cancer, which comprises the administration of the medicament or said pharmaceutical composition of this invention that contains a compound of this invention. Such medicament or composition is administered to a mammal, preferably human, by any route that may effectively transport the active ingredient(s) of the composition to the appropriate or desired site of action, such as oral, nasal, pulmonary, transdermal, such as passive or iontophoretic delivery, or parenteral, e.g., rectal, depot, subcutaneous, intravenous, intramuscular, intranasal, ophthalmic solution or an ointment. Further, the administration of the compound of this invention with other active ingredients may be concurrent or simultaneous.

It will be appreciated that the dosage of compounds of the present invention will vary from patient to patient not only for the particular compound or composition selected, the route of administration, and the ability of the compound (alone or in combination with one or more drugs) to elicit a desired response in the patient, but also factors such as disease state or severity of the condition to be alleviated, age, sex, weight of the patient, the state of being of the patient, and the severity of the pathological condition being treated, concurrent medication or special diets being followed by the patient, and other factors which those skilled in the art will recognize, with the appropriate dosage ultimately being at the discretion of the attendant physician. Dosage regimens may be adjusted to provide the improved therapeutic response. A therapeutically effective amount is also one in which any toxic or detrimental effects of the compound or composition are outweighed by the therapeutically beneficial effects. Preferably, the compounds or compositions of the present invention are administered at a dosage and for a time such that the number and/or severity of the symptoms are decreased.

Finally, the synthesis of any of the compounds of the invention is clearly described in the examples of the present invention.

In this sense, a further embodiment of the invention refers to a method or procedure for the synthesis of glycomimetic isothiocyanates, such as compounds **13β, 14β, 14α, 15β** and **15α** comprising the following steps:
a) To a solution of the corresponding azide of the glycomimetic compound in a suitable solvent such as diethyl ether, adding Triphenylphosphine (PPh₃), preferably at room temperature and keeping stirring under reflux;
b) Once the starting product is consumed (preferably monitoring by TLC) the solvent is evaporated, preferably under reduced pressure;
c) Dissolving the product of b) in carbon disulfide (CS₂), preferably stirring at reflux until no evolution of the reaction (monitoring by TLC); and
d) Then, preferably the excess of CS₂ is evaporated under reduced pressure, and the product of c) is preferably purified, more preferably by flash column chromatography.

A further embodiment of the invention refers to a method or procedure for the synthesis of Methyl 2,4,6-tri-*O*-acetyl-3-isothiocyanato-1,3-dideoxy-1-thio-β-D-glucopyranoside, **13β**, comprising the following steps:
a) The reaction is carried out starting from **10β** in a suitable solvent such as diethyl ether (Et₂O), by adding PPh₃;
b) Once the starting product is consumed (preferably by monitoring by TLC) the solvent is evaporated, preferably under reduced pressure;
c) Dissolving the product of b) in carbon disulfide (CS₂), preferably stirring at reflux until no evolution of the reaction (monitoring by TLC); and
d) Then, preferably the excess of CS₂ is evaporated under reduced pressure, and the product of c) is preferably purified, more preferably by flash column chromatography.

A further embodiment of the invention refers to a method or procedure for the synthesis of Ethyl 2,4,6-tri-*O*-acetyl-3-isothiocyanato-1,3-dideoxy-1-thio-β-D-glucopyranoside, **14β**, comprising the following steps:
a) The reaction is carried out starting from **11β** in a suitable solvent such as diethyl ether (Et₂O), by adding PPh₃;
b) Once the starting product is consumed (preferably by monitoring by TLC) the solvent is evaporated, preferably under reduced pressure;
c) Dissolving the product of b) in carbon disulfide (CS₂), preferably stirring at reflux until no evolution of the reaction (monitoring by TLC); and
d) Then, preferably the excess of CS₂ is evaporated under reduced pressure, and the product of c) is preferably purified, more preferably by flash column chromatography.

A further embodiment of the invention refers to a method or procedure for the synthesis of Phenyl 2,4,6-tri-*O*-acetyl-3-isothiocyanato-1,3-dideoxy-1-thio-β-D-glucopyranoside, **15β**, comprising the following steps:
a) The reaction is carried out starting from **12β** in a suitable solvent such as diethyl ether (Et₂O), by adding PPh₃;
b) Once the starting product is consumed (preferably by monitoring by TLC) the solvent is evaporated, preferably under reduced pressure;
c) Dissolving the product of b) in carbon disulfide (CS₂), preferably stirring at reflux until no evolution of the reaction (monitoring by TLC); and
d) Then, preferably the excess of CS₂ is evaporated under reduced pressure, and the product of c) is preferably purified, more preferably by flash column chromatography.

A further embodiment of the invention refers to a method or procedure for the synthesis of Ethyl 2,4,6-tri-O-acetyl-3-isothiocyanato-1,3-dideoxy-1-thio-α-D-glucopyranoside, **14α,** comprising the following steps:
a) The reaction is carried out starting from **11α** in a suitable solvent such as diethyl ether (Et₂O), by adding PPh₃;
b) Once the starting product is consumed (preferably by monitoring by TLC) the solvent is evaporated, preferably under reduced pressure;
c) Dissolving the product of b) in carbon disulfide (CS₂), preferably stirring at reflux until no evolution of the reaction (monitoring by TLC); and
d) Then, preferably the excess of CS₂ is evaporated under reduced pressure, and the product of c) is preferably purified, more preferably by flash column chromatography.

A further embodiment of the invention refers to a method or procedure for the synthesis of Phenyl 2,4,6-tri-O-acetyl-3-isothiocyanato-1,3-dideoxy-1-thio-α-D-glucopyranoside, **15α,** comprising the following steps:
a) The reaction is carried out starting from **12α** in a suitable solvent such as diethyl ether (Et₂O), by adding PPh₃;
b) Once the starting product is consumed (preferably by monitoring by TLC) the solvent is evaporated, preferably under reduced pressure;
c) Dissolving the product of b) in carbon disulfide (CS₂), preferably stirring at reflux until no evolution of the reaction (monitoring by TLC); and
d) Then, preferably the excess of CS₂ is evaporated under reduced pressure, and the product of c) is preferably purified, more preferably by flash column chromatography.

A still further embodiment of the invention refers to a method or procedure for the synthesis of glycomimetic thioderivatives, such as compounds (*S*_{S})-**16β**, (*S*_{S})-**17β**, (*R*_{S})-**17β**, , (*S*_{S})-**18β**, (*R*_{S})-**18β**, (*R*ₛ)-**17α**, (*R*ₛ)-**18α**, comprising the following steps:
a) To a solution of the corresponding thioderivative of the glycomimetic compound in a suitable solvent such as dichloromethane in an inert atmosphere, adding a solution of meta-Chloroperoxybenzoic acid (*m*-CPBA) in a suitable solvent, such as trichloromethane, preferably at a reaction temperature of about -78 ºC;
b) Once the starting product is consumed (preferably by monitoring by TLC) the reaction is extracted with saturated NaHCO₃ solution, preferably washed with saturated NaCl solution, preferably dried over anhydrous Na₂SO₄, preferably filtered and the solvent is evaporated, preferably under reduced pressure;
c) Then, the product of b) is preferably purified, more preferably by flash column chromatography.

It is noted that:
- Compound Methyl 2,4,6-tri-*O*-acetyl-3-isothiocyanato-1,3-dideoxy-1-(*S*)-sulfinyl-β-D-glucopyranoside, (*S*ₛ)-**16β** is produced by the above reaction starting from **13β**;
- Compound Ethyl 2,4,6-tri-*O*-acetyl-3-isothiocyanato-1,3-dideoxy-1[(*S*)-sulfinyl]-β-D-glucopyranose, (*S*ₛ)-**17β** is produced by the above reaction starting from **14β**;
- Compound Ethyl 2,4,6-tri-O-acetyl-3-isothiocyanato-1,3-dideoxy-1[(*R*)-sulfinyl]-β-D-glucopyranose, (*R*ₛ)-**17β** is produced by the above reaction starting from **14β**;
- Compound Phenyl 2,4,6-tri-O-acetyl-3-isothiocyanato-1,3-dideoxy-1[(*S*)-sulfinyl]-β-D-glucopyranose, (*S*ₛ)-**18β** is produced by the above reaction starting from **15β**;
- Compound Phenyl 2,4,6-tri-O-acetyl-3-isothiocyanato-1,3-dideoxy-1[(*R*)-sulfinyl]-β-D-glucopyranose, (*R*ₛ)-**18β** is produced by the above reaction starting from **15β**;
- Compound Ethyl 2,4,6-tri-O-acetyl-3-isothiocyanato-1,3-dideoxy-1[(*S*)-sulfinyl]-α-D-glucopyranse, (*R*ₛ)-**17α** is produced by the above reaction starting from **14α**; and
- Compound Phenyl 2,4,6-tri-O-acetyl-3-isothiocyanato-1,3-dideoxy-1[(*S*)-sulfinyl]-α-D-glucopyranose, (*R*ₛ)-**18α** is produced by the above reaction starting from **15α**.

A still further embodiment of the invention refers to a method or procedure for the synthesis of glycomimetic thioderivatives (by proceeding to the oxidation of a thioether to sulfone), such as compounds **19β**, **20α, 21α, 20β,** and **21β,** comprising the following steps:
a) To a solution of the corresponding thioderivative of the glycomimetic compound in a suitable solvent such as dichloromethane in an inert atmosphere, adding a solution of meta-chloroperoxybenzoic acid (*m*-CPBA) in a suitable solvent, such as trichloromethane, preferably at room temperature;
b) Once the starting product is consumed (preferably by monitoring by TLC) the reaction is extracted with saturated NaHCO₃ solution, preferably washed with saturated NaCl solution, preferably dried over anhydrous Na₂SO₄, preferably filtered and the solvent is evaporated, preferably under reduced pressure;
c) Then, the product of b) is preferably purified, more preferably by flash column chromatography.

It is noted that:
- Compound Methyl 2,4,6-tri-O-acetyl-3-isothiocyanato-1,3-dideoxy-1-sulfonyl-β-D-glucopyranose, **19β** is produced by the above reaction starting from **13β**;
- Compound Ethyl 2,4,6-tri-O-acetyl-3-isothiocyanato-1,3-dideoxy-1-sulfonyl-β-D-glucopyranose, **20β** is produced by the above reaction starting from **14β**;
- Compound Phenyl 2,4,6-tri-O-acetyl-3-isothiocyanato-1,3-dideoxy-1-sulfonyl-β-D-glucopyranose, **21β** is produced by the above reaction starting from **15β**;
- Compound Ethyl 2,4,6-tri-O-acetyl-3-isothiocyanato-1,3-dideoxy-1-sulfonyl-α-D-glucopyranose, **20α** is produced by the above reaction starting from **14α**; and
- Compound Phenyl 2,4,6-tri-O-acetyl-3-isothiocyanato-1,3-dideoxy-1-sulfonyl-α-D-glucopyranose, **21α** is produced by the above reaction starting from **15α**.

In the context of this disclosure, a number of terms shall be used.

The term "treating" or "treatment" as used herein refers to administering a compound of this invention to arrest the growth of at least 45%, 50%, 55%, 60% or 65% of the cancerous cells at G2/M cycle, preventing them from multiplying, and hence results in the reduction of the size of the cancer. Therefore, the term "treating" or "treatment" as used herein also refers to kill or induce apoptosis of the cancerous cells.

The term "an effective amount" as used herein refers to an amount effective, at dosages, and for periods of time necessary, to achieve the desired therapeutically desired result with respect to the treatment of cancer.

The terms "compounds", "compositions", "active compounds", "agent" or "medicament" are used interchangeably herein to refer to a compound or compounds or composition of which, when administered to a subject (human or animal) induces a desired pharmacological and/or physiological effect by local and/or systemic action.

The term "administered", "administering" or "administration" are used interchangeably herein to refer means either directly administering a compound or a composition of the present invention, or administering a prodrug, derivative or analogue which will form an equivalent amount of the active compound within the body.

The term "subject" or "patient" refers to an animal including the human species that is treatable with the compositions and/or methods of the present invention. The term "subject" or "patient" intended to refer to both the male and female gender unless one gender is specifically indicated. Accordingly, the term "subject" or "patient" comprises any mammal, preferably a human, which may benefit from treatment by the compound of this disclosure.

The present invention will now be described more specifically with reference to the following embodiments, which are provided for the purpose of demonstration rather than limitation.

### Examples

### Example 1.

The following tables 1-3 show the results obtained from the activity studies conducted with the different isothiocyanates included in the Markush formula below:

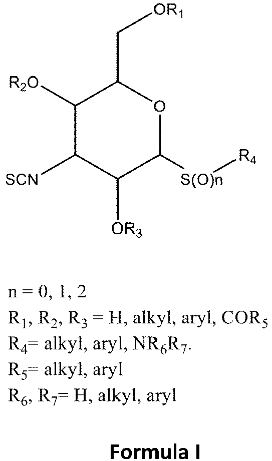

SFN and compounds **4-6** correspond to the natural isothiocyanates sulforaphane, iberverin, iberin and cheirolin respectively, which are used as reference. Compounds **13-21** correspond to the isothiocyanate carbohydrate-based derivatives of the present invention.

Activation capacity of the NrF2 factor is shown in table 1 below:

### NrF2 activation

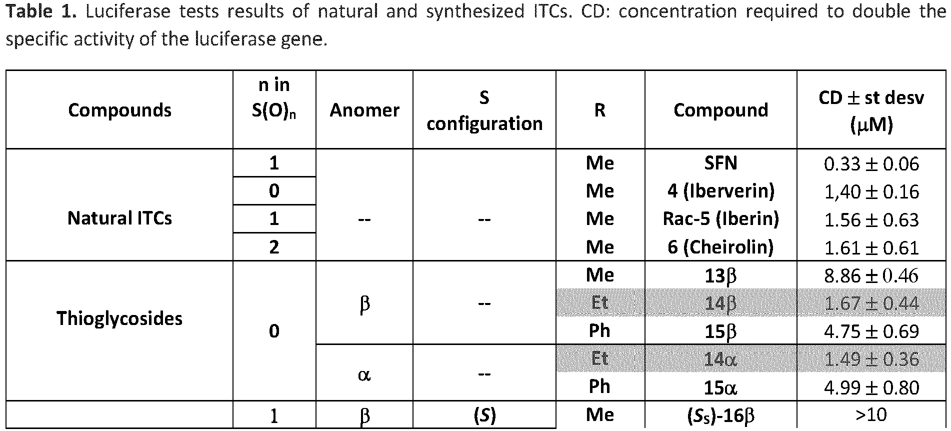

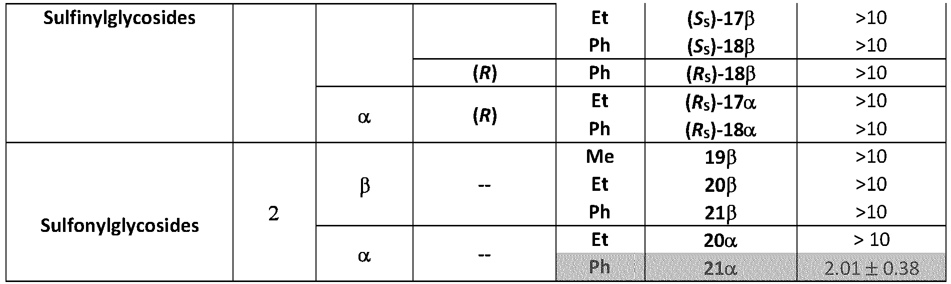

As shown in table 2 above, the best results were obtained for the following chemical compounds:

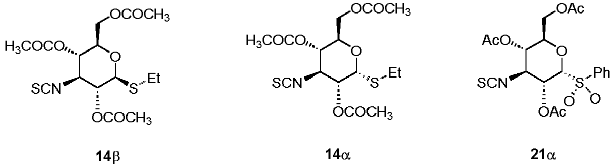

Cytotoxic activity against solid tumours is shown in Table 2 below:

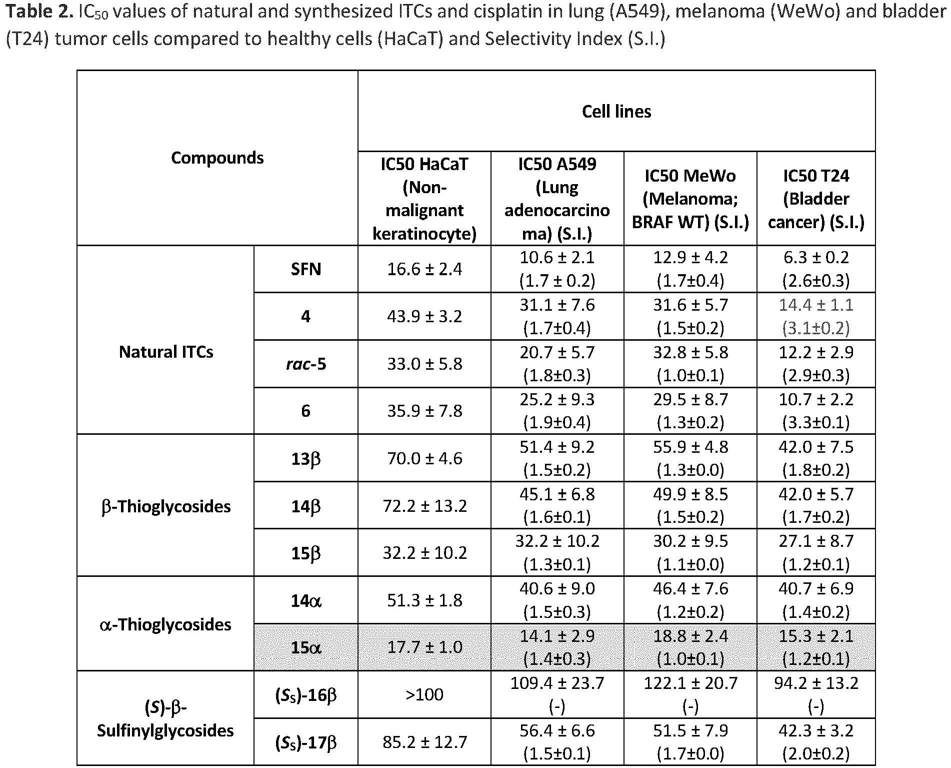

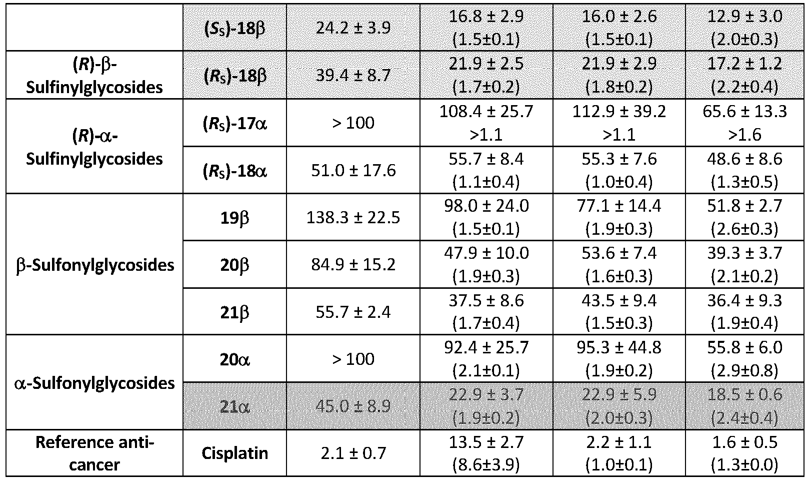

As shown in table 2 above, the best results were obtained for the following chemical compounds: 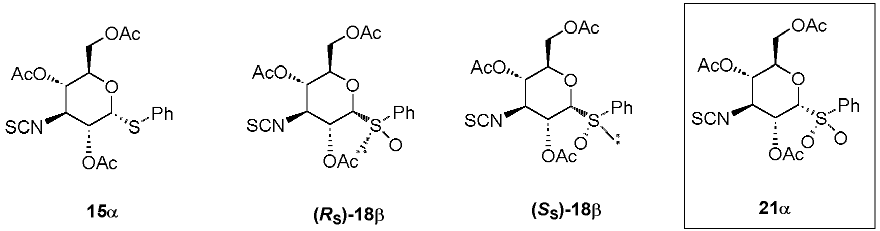 and antileukemic activity is shown in Table 3 below:

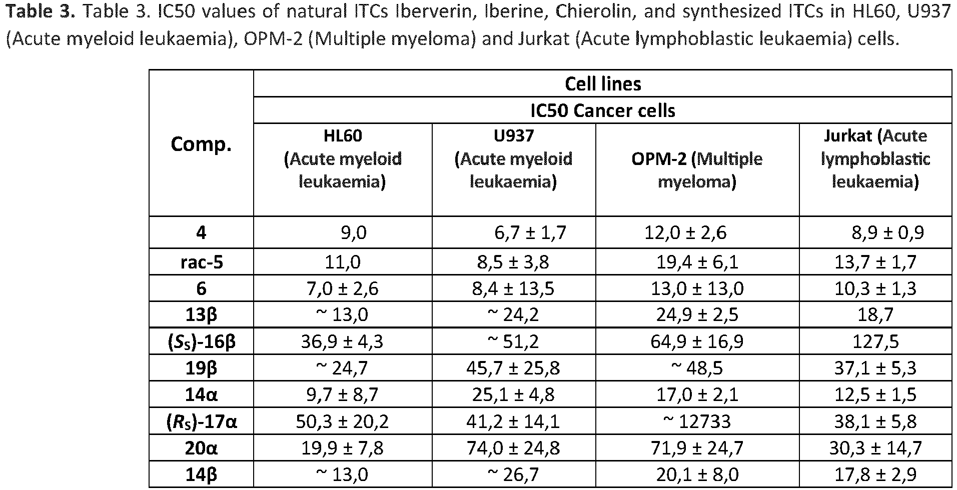

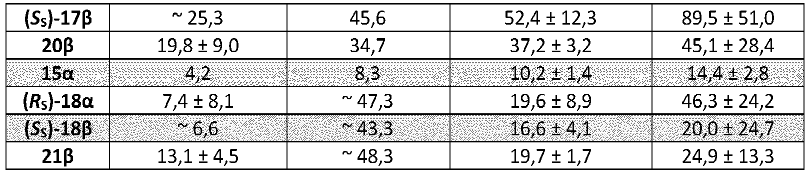

As shown in table 3 above, the best results were obtained for the following chemical compounds:

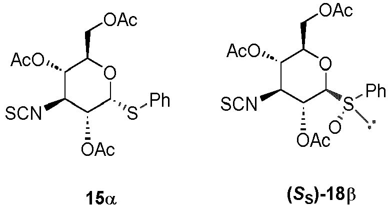

### Example 2. Material and methods and Synthesis

The synthetic route followed for the preparation of the compounds exemplified herein is represented in Scheme 1 below, where reaction conditions and yields are indicated.

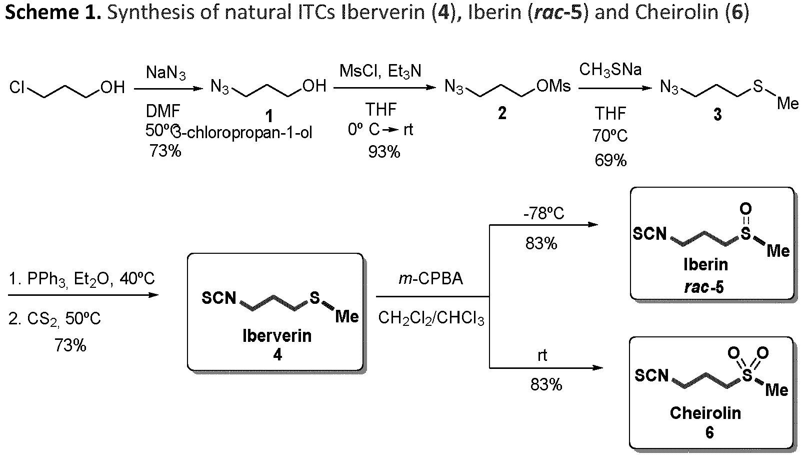

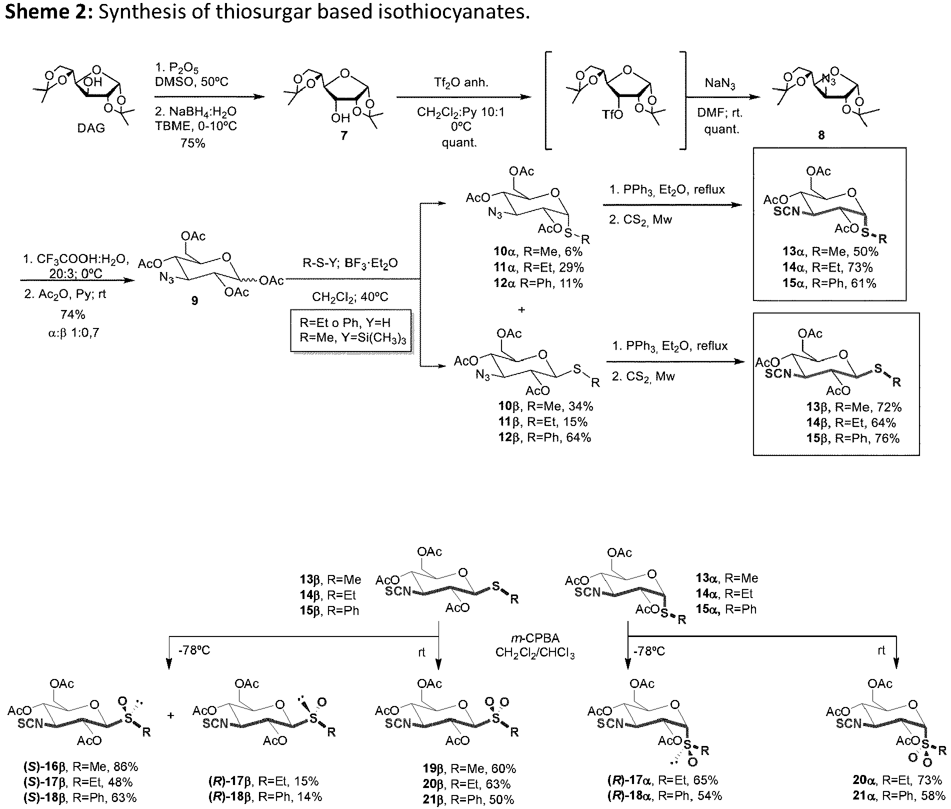

Anhydrous solvents were purchased from the Sharlau company and have been dried on an activated molecular sieve, thus providing anhydrous dimethylformamide (DMF), tert-butylmethyl ether (TBME) and dichloromethane (CH₂Cl₂). In cases where anhydrous solvents were not required, they were used with an analytical purity degree.

Commercial reagents were used without any further purification.

Thin layer chromatography was carried out using 0.25 nm Alufram^{®}Sil.G/V245 Merck aluminum-supported silica gel chromatoplates, and the development of the eluted and dried plates was carried out with sulfuric acid. The purification of the compounds was carried out generally in a chromatographic column under pressure, Merck silica gel 60 Å (pore size 40-63 µm) was used as the stationary phase. The composition of the eluent used is detailed for each compound.

Nuclear Magnetic Resonance (¹H-NMR, ¹³C-NMR) was recorded with Bruker AMX-500 and Bruker Advance DRX-500 (500 MHz) spectrometers from the Center for Research, Technology and Innovation of the University of Seville, CITIUS. The spectra were recorded from samples in solution in the deuterated solvent indicated in parentheses. Chemical shifts (δ) are expressed in ppm, using the residual signal of the non-deuterated solvent as a reference.

High resolution mass spectra (HRMS) was carried out by the Mass Spectrometry Service of the University of Seville on a Kratos MS-80-RFA spectrometer and on a Micromass AutoSpec model mass spectrometer.

### 3-Azidopropan-1-ol, 1

To a solution of 3-chloropropan-1-ol (10.00 g, 105.77 mmol) in DMF (100 mL), NaN₃ (13.75 g, 211.54 mmol) is added at room temperature. After 24 hours stirring at 50°C, the reaction mixture is hydrolysed with distilled H₂O and the DMF is evaporated under reduced pressure. The crude is dissolved in CH₂Cl₂ and washed with distilled H₂O (1 × 40 mL) and saturated NaCl solution (1 × 40 mL). The organic phase is dried over Na₂SO₄ and filtered. The solvent is evaporated under reduced pressure to obtain 7.81 g, 77.20 mmol (73% yield) of 1 as a yellow liquid. ¹H-NMR (500 MHz, CDCl₃): δ 3.75 (t, *J* = 6.0 Hz, 2H, - OCH₂), 3.44 (t, *J* = 6.6 Hz, 2H, N₃CH₂), 1.83-1.74 (m, 2H, -CH₂CH₂CH₂-), 1.69 (s, 1H, OH) ppm. ¹³C-NMR (125 MHz, CDCl₃): δ 60.1, 48.6, 31.6 ppm. HRMS Calcd for C₃H₇ON₃Na [M+Na]+: 124.0481; found 124.0478 (-2.3312 ppm).

### 3-azidopropyl methanesulfonate, 2

To a solution of **1** (1.12 g, 11.11 mmol) in THF (10 mL) under argon atmosphere and at 0 °C is added Et₃N (1.85 mL, 13 0.33 mmol) and subsequently MsCI (1.03 mL, 13.33 mmol). Once the reaction is complete (12 hours), the solvent is evaporated under reduced pressure. The residue is dissolved in CH₂Cl₂ and washed with saturated NH₄Cl solution (1 × 20 mL) and with saturated NaCl solution (1 × 20 mL). The organic solution is dried over anhydrous Na₂SO₄, filtered and the solvent is evaporated under reduced pressure to obtain 1.86 g, 10.37 mmol (93% yield) of **2** as an orange liquid. ¹H-NMR (500 MHz, CDCl₃): δ 4.31 (t, *J* = 6.0 Hz, 2H, - OCH₂-), 3.47 (t, *J* = 6.5 Hz, 2H, -CH₂N₃), 3.00 (s, 3H, -SCH₃), 2.00 (quint, *J* = 6.2 Hz, 2H, - CH₂CH₂CH₂-) ppm. ¹³C-NMR (125 MHz, CDCl₃): δ 66.6, 47.5, 37.5, 28.8 ppm. HRMS Calcd for C₄H₉O₃N₃NaS [M+Na]+: 202.0257; found 202.0252 (-2.5 ppm).

### (3-azidopropyl)(methyl)sulfane, 3

To a solution of **2** (1.86 g, 10.37 mmol) in THF (40 mL), CH₃SNa (1.47 g, 20.74 mmol) is added under an argon atmosphere. After stirring at 70 °C for 12 hours, the reaction is allowed to cool to room temperature and washed with saturated NaCl solution (1 × 20 mL). The mixture is dried over anhydrous Na₂SO₄, filtered and the solvent is evaporated under reduced pressure to obtain 0.94 g, 7.17 mmol (69% yield) of **3** as a yellow liquid. ¹H-NMR (500 MHz, CDCl₃): δ 3.41 (t, *J* = 6.6 Hz, 2H, - OCH₂-), 2.57 (t, *J* = 7.1 Hz, 2H, -CH₂N₃), 2.10 (s, 3H, -SCH₃), 1.87 (quint, *J* = 6.9 Hz, 2H, - CH₂CH₂CH₂-) ppm. ¹³C-NMR (125 MHz, CDCl₃): δ 50.2, 31.3, 28.4, 15.7 ppm.

### 1,2,5,6-O-isopropylidene-α-D-allofuranose, 7

To 25 mL of DMSO under argon atmosphere and at 18-20 °C, P₂O₅ (5.45 g, 38.42 mmol) is slowly added. After stirring for 15 minutes at 18-20 °C, a solution of DAG (10.00 g, 38.42 mmol) in 50 mL of DMSO is added. The mixture is then heated at 50 °C until the starting product is consumed (3 hours). Next, after adding TBME (60 mL) to the mixture, both phases are separated, and the aqueous phase is extracted with TBME (40 mL). The combined organic phases are evaporated under reduced pressure to a volume of 75 mL and added, at 0-10 °C, to a solution of NaBH₄ (0.92 g, 24.20 mmol) in distilled H₂O (38 mL). After stirring for 30 minutes, the reaction mixture is diluted with distilled H₂O (40 mL), both phases are separated, the aqueous phase is extracted with CH₂Cl₂ (3 × 40 mL) and the combined organic phases are evaporated under reduced pressure. The obtained crude is dissolved in TBME (75 mL) and extracted with distilled H₂O (3 × 30 mL). The combined aqueous phases are extracted with CH₂Cl₂ (5 × 40 mL) and the organic extracts are dried over anhydrous Na₂SO₄. The solvent is evaporated under reduced pressure to obtain 9.92 g, 38.11 mmol (quant. yield) of **7** as a white solid. ¹H-NMR (500 MHz, CDCl₃): 5.81 (d, *J* = 3.8 Hz, 1H, H₁), 4.61 (dd, *J* = 3.9 and 5.1 Hz, 1H, H₄), 4.30 (td, *J* = 4.8 and 6.6 Hz, 1H, H₅), 4.10-3.99 (m, 3H, H2, H₆ and H_{6'}), 3.82 (dd, *J* = 4.8 and 8.5 Hz, 1H, H₃), 2.52 (d, *J* = 8.4 Hz, 1H, OH), 1.58 (s, 3H, CH₃), 1.46 (s, 3H, CH₃), 1.38 (s, 3H, CH₃), 1.37 (s, 3H, CH₃) ppm. ¹³C-NMR (125 MHz, CDCl₃): δ 113.0, 110.0, 104.1, 80.0, 79.2, 75.8, 72.7, 66.1, 26.7 (2C), 26.5, 25.4 ppm. HRMS: Calcd for C₁₂H₂₀O₆Na [M+Na]+: 283.1152; found 283.1156 (0.4 ppm).

### 3-Deoxy-1,2,5,6-O-isopropylidene-3-azido-α-D-glucofuranose, 8

To a solution of **7** (5.00 g, 19.21 mmol) in a CH₂Cl₂:Pyridine 10:1 mixture (55 mL), under argon atmosphere and at 0 °C, trifluoromethanesulfonic anhydride (4.82 mL, 28.81 mmol) is added dropwise. After stirring for 30 minutes, the starting product is consumed, and the reaction mixture is quenched with distilled H₂O. The aqueous phase is extracted with CH₂Cl₂ (3 × 50 mL) and the combined organic phases are washed with saturated NaCl solution (1 × 30 mL), dried over anhydrous Na₂SO₄ and filtered. The solvent is evaporated under reduced pressure. To a solution of the obtained triflate in DMF (200 mL), NaN₃ (5.77 g, 88.83 mmol) is added at 0 °C and the temperature is allowed to rise to room temperature. Once the starting product is consumed (24 hours), the reaction is quenched with distilled H₂O and the solvent is evaporated under reduced pressure. The residue is dissolved in saturated NaCl solution and extracted with CH₂Cl₂ (3 × 50 mL). The combined organic phases are washed with saturated NaCl solution (1 × 30 mL), dried over anhydrous Na₂SO₄, filtered, and the solvent is evaporated under reduced pressure to obtain 5.06 g, 17.73 mmol (quant. yield) of **8** as an orange syrup. ¹H-NMR (500 MHz, CDCl₃): δ 5.85 (d, *J* = 3.6 Hz, 1H, H₁), 4.61 (d, *J* = 3.6 Hz, 1H, H₄), 4.26 - 4.21 (m, 1H, H₅), 4.15 - 4.08 (m, 3H, H₂, H₆ and H_{6'}), 3.97 (dd, *J* = 8.7 and 4.9 Hz, 1H, H₃), 1.51 (s, 3H, CH₃), 1.43 (s, 3H, CH₃), 1.37 (s, 3H, CH₃), 1.32 (s, 3H, CH₃) ppm. ¹³C-NMR (125 MHz, CDCl₃): δ 112.5, 109.7, 105.2, 83.6, 80.7, 73.2, 67.8, 66.6, 27.0, 26.8, 26.4, 25.3 ppm. HRMS: Calcd for C₁₂H₁₉O₅N₃Na [M+Na]+: 308.1217; found 308.1219 (0.7 ppm).

### 3-Azido-3-deoxy-α,β-D-glucopyranose tetraacetate, 9

Compound **8** (5.03 g, 17.65 mmol) is dissolved in a mixture of CF₃COOH:H₂O 20:3 (43 mL) at 0°C. After stirring for 1 hour, the starting product is consumed, and the solvent is evaporated under reduced pressure. To a solution of the crude in pyridine (150 mL) under argon atmosphere and at 0 °C, acetic anhydride (81.70 mL, 864.75 mmol) is added and the temperature is allowed to rise to room temperature. After stirring for 18 hours, the reaction is quenched with ice-water and this mixture is extracted with CH₂Cl₂ (3 × 30 mL). Next, the organic phase is washed with 2 M H₂SO₄ solution (3 × 30 mL), saturated NaHCO₃ solution (3 × 20 mL) and saturated NaCl solution (1 × 20 mL). The mixture is dried over anhydrous Na₂SO₄, filtered and the solvent is evaporated under reduced pressure. The crude is purified by flash column chromatography (hexane/AcOEt 5:1) to obtain 4.86 g, 13.02 mmol (74% yield) of **9** as a mixture of the two possible α:β anomers in a ratio of 1: 0.7, as a yellow oil. ¹H-NMR (500 MHz, CDCl₃): δ 6.29 (d, *J* = 3.6 Hz, 1Hα, H₁), 5.66 (d, *J* = 8.3 Hz, 1HP, H₁), 5.05-4,98 (m, 1Hα, H₆, 2Hβ, H₆ and H_{6'}), 4.94 (dd, *J* = 3.6 and 10.7 Hz, 1Hα, H_{6'}), 4.25-4.18 (m, 1Hα, H₄, 1Hβ, H₄), 4.08 (td, *J* = 2.1 and 13.0 Hz, 1Hα, H₅), 4.06-4.00 (m, 1Hα, H₂, 1Hβ, H₂), 3.95 (t, *J* = 10.3 Hz, 1Hα, H₃), 3.78 (ddd, *J* = 2.3, 4.7 and 9.9 Hz 1HP, H₅), 3.68 (t, *J* = 10.1 Hz, 1HP, H₃), 2.17 (s, 3Hα, CH₃), 2.13 (s, 3Hα, CH₃), 2.12 (s, 3Hβ, CH₃), 2.11 (s, 3Hβ, CH₃), 2.10 (s, 3Hβ, CH₃), 2.09-2.06 (m, 6Hα, 3Hβ, CH₃) ppm. ¹³C-NMR (125 MHz, CDCl₃): δ 170.7, 170.7, 169.5, 169.2, 169.2, 169.1, 169.0, 168.7, 92.1, 88.9, 73.7, 70.3, 70.2, 70.1, 68.0, 68.0, 64.4, 61.7, 61.6, 61.0, 20.9, 20.9, 20.8, 20.7, 20.7, 20.6 ppm. HRMS: Calcd for C₁₄H₁₉O₉N₃Na [M+Na]+: 396.1014; found 396.1008 (-1.3 ppm).

### Methyl 2,4,6-tri-O-acetyl-3-azido-1,2-dideoxy-1-thio-β-D-glucopyranoside, 10β

To a solution of compound 9 (0.43 g, 1.15 mmol) in CH₂Cl₂ (4.2 mL) are added dropwise under argon atmosphere and at room temperature trimethyl(methylthio)silane (0.41 mL, 2.87 mmol) and subsequently boron trifluoride etherate (1.16 mL, 9.18 mmol). The mixture is kept under stirring at 40 °C. After 17 hours, the reaction mixture is quenched with saturated NaHCO₃ solution (15 mL). The aqueous phase is extracted with CH₂Cl₂ (3 × 25 mL) and the combined organic phases are washed with saturated NaCl solution (1 × 25 mL), dried over anhydrous Na₂SO₄, filtered, and the solvent is evaporated under reduced pressure. The crude is purified by flash column chromatography (hexane/AcOEt 7:1) to obtain 0.138 g, 0.38 mmol (34% yield) of **1013** as a brown syrup. ¹H-NMR (500 MHz, CDCl₃): δ 4.97 - 4.93 (m, 2H, H₂ and H₄), 4.32 (d, *J* = 9.8 Hz, 1H, H₁), 4.19 (dd, *J* = 4.9 and 12.4 Hz, 1H, H₆), 4.11 (dd, *J* = 2.4 and 12.4 Hz, 1H, H_{6'}), 3.68 - 3.64 (m, 2H, H₃ and H₅), 2.14 (s, 3H, CH₃COO-), 2.13 (s, 3H, CH₃COO-), 2.11 (s, 3H, CH₃COO-), 2.06 (s, 3H, -SCH₃) ppm. ¹³C-NMR (125 MHz, CDCl₃): δ 170.7, 169.3 (2), 83.2, 76.7, 69.3, 68.4, 65.8, 62.2, 20.8, 20.1, 11.2 ppm. HRMS: Calcd for C₁₃H₁₉O₇N₃NaS [M+Na]+: 384.0828; found 384.0836 (-0.1 ppm).

### General procedure for the synthesis of ethyl and phenylthioglycosides

To a solution of compound 9 (100 mol%) in CH₂Cl₂ are added dropwise under argon atmosphere and at room temperature, the corresponding thiol (200 mol%) and subsequently boron trifluoride etherate (400 mol%). The mixture is kept under stirring at 40 °C. Once the starting product is consumed (17 h), the reaction mixture is treated with saturated NaHCO₃ solution. The aqueous phase is extracted with CH₂Cl₂ (3 times) and the combined organic phases are washed with saturated NaCl solution, dried over anhydrous Na₂SO₄, filtered and the solvent is evaporated under reduced pressure.

### Ethyl 2,4,6-tri-O-acetyl-3-azido-1,2-dideoxy-1-thio-α and β-D-glucopyranoside

The reaction is carried out following the general procedure starting from compound **9** (1.50 g, 4.02 mmol), in CH₂Cl₂ (15 mL), ethanethiol (0.60 mL, 8.04 mmol) and boron trifluoride etherate (1.89 mL, 16.08 mmol). In this way, a mixture of the two α:β anomers in a 1:1 ratio is obtained. After purification by flash column chromatography (hexane/AcOEt 7:1), the α-anomer **11**α (0.376 g, 1.06 mmol) is obtained as a brown syrup and the β-anomer **11β** (0.284 g, 0.76 mmol) is obtained as a yellow syrup, with an overall yield of 44% (25% **11α** and 19% **11β)**.

### Ethyl 2,4,6-tri-O-acetyl-3-azido-1,2-dideoxy-1-thio-β-D-glucopyranoside, 11β

¹H-NMR (500 MHz, CDCl₃): δ 4.97 - 4.92 (m, 2H, H₂ and H₄), 4.43 (d, *J* = 9.9 Hz, 1H, H₁), 4.19 (dd, *J* = 5.1 and 12.4 Hz, 1H, H₆), 4.11 (dd, *J* =2.5 and 12.3 Hz, 1H, H_{6'}), 3.67 - 3.63 (m, 2H, H₅ and H₃), 2.75 - 2.63 (m, 2H, -CH₂CH₃), 2.13 (s, 3H, CH₃COO-), 2.11 (s, 3H, CH₃COO-), 2.07 (s, 3H, CH₃COO-), 1.25 (t, *J* = 7.5 Hz, 3H, -CH₂CH₃) ppm. ¹³C-NMR (125 MHz, CDCl₃): δ 170.8, 169.3 (2), 83.9, 76.6, 70.1, 68.5, 65.9, 62.3, 24.1, 20.9 (2), 20.8, 14.9 ppm. HRMS: Calcd for C₁₄H₂₁O₇N₃NaS [M+Na]+: 398.0992; found 398.0992 (0.0 ppm).

### Ethyl 2,4,6-tri-O-acetyl-3-azido-1,2-dideoxy-1-thio-α-D-glucopyranoside, 11α

¹H-NMR (500 MHz, CDCl₃): δ 5.65 (d, *J* = 5.7 Hz, 1H, H₁), 4.90 (t, *J* = 10.0 Hz, 1H, H₄), 4.87 (dd, *J* = 5.6 and 10.6 Hz, 1H, H₂), 4.34 (ddd, *J* = 2.3, 4.8 and 10.1 Hz, 1H, H₅), 4.22 (dd, *J* = 4.9 and 12.3 Hz, 1H, H₆), 4.04 (dd, *J* = 2.3 and 12.4 Hz, 1H, H_{6'}), 3.86 (t, *J* = 10.2 Hz, 1H, H₃), 2.61- 2.48 (m, 2H, -CH₂CH₃), 2.12 (s, 3H, CH₃COO-), 2.11 (s, 3H, CH₃COO-), 2.06 (s, 3H, CH₃COO-), 1.26 (t, *J* =7.4 Hz, 3H, -CH₂CH₃) ppm. ¹³C-RMN (125 MHz, CDCl₃): δ 170.7, 169.7, 169.4, 81.7, 71.8, 68.5, 67.8, 62.1, 62.0, 24.3, 20.9, 20.8, 20.7, 14.8 ppm. HRMS: Calcd for C₁₄H₂₁O₇N₃NaS [M+Na]+: 398.0992; found 398.0992 (0.0 ppm).

### Phenyl 2,4,6-tri-O-acetyl-3-azido-1,2-dideoxy-1-thio-α and β-D-glucopyranoside

The reaction is carried out following the general procedure starting from compound **9** (3.30 g, 8.84 mmol), in CH₂Cl₂ (25 mL), thiophenol (1.80 mL, 17.68 mmol) and boron trifluoride etherate (4.48 mL, 35.36 mmol). In this way, a mixture of the two α:β anomers in a 0.2:1 ratio is obtained. After purification by flash column chromatography (hexane/AcOEt 7:1), the α-anomer **12α** (0.416 g, 1.06 mmol) is obtained as a brown syrup and the β-anomer **12β** (2.38 g, 5.62 mmol) is obtained as a yellow syrup, with an overall yield of 75% (11% **12α** and 64% **12β**).

### Phenyl 2,4,6-tri-O-acetyl-3-azido-1,2-dideoxy-1-thio-β-D-glucopyranoside, 12β

¹H-NMR (500 MHz, CDCl₃): δ 7.51 - 7.49 (m, 2H, SC₆H₅), 7.33 - 7.30 (m, 3H, SC₆H₅), 4.93 (t, *J* = 10.0 Hz, 1H, H₂), 4.91 (t, *J* = 9.9 Hz, 1H, H₄), 4.66 (d, *J* = 9.9 Hz, 1H, H₁), 4.21 - 4.14 (m, 2H, H6, H_{6'}), 3.69 - 3.64 (m, 2H, H₅, H₃), 2.18 (s, 3H, CH₃COO-), 2.12 (s, 3H, CH₃COO-), 2.08 (s, 3H, CH₃COO-) ppm. ¹³C-NMR (125 MHz, CDCl₃): δ 170.7, 169.3, 169.2, 133.1, 132.1, 129.1, 128.5, 86.5, 76.6, 70.2, 68.5, 66.0, 62.4, 20.9, 20.8, 20.7 ppm. HRMS: Calcd for C₁₈H₂₁O₇N₃NaS [M+Na]+: 446.0992; found 446.0987 (-1,3 ppm). M.p.: 117-119 °C. [α]_{D}²⁰:-18,2 (c 1, CHCl₃).

### Phenyl 2,4,6-tri-O-acetyl-3-azido-1,2-dideoxy-1-thio-α-D-glucopyranoside, 12α

¹H-NMR (500 MHz, CDCl₃): δ 7.44 - 7.42 (m, 2H, SC₆H₅), 7.33 - 7.28 (m, 3H, SC₆H₅), 5.89 (d, *J* = 5.6 Hz, 1H, H₁), 4.96 (dd, *J* = 5.4 and 10.6 Hz, 1H, H₂), 4.95 (t, *J* = 9.9 Hz, 1H, H₄), 4.49 (ddd, *J* = 2.3, 5.3 and 10.1 Hz, 1H, H₅), 4.22 (dd, *J* = 5.4 and 12.4 Hz, 1H, H₆), 4.02 (dd, *J* = 2.3 and 12.4 Hz, 1H, H_{6'}), 3.94 (t, *J* = 10.3 Hz, 1H, H₃), 2.18 (s, 3H, CH₃COO-), 2.15 (s, 3H, CH₃COO-), 2.01 (s, 3H, CH₃COO-) ppm. ¹³C-NMR (125 MHz, CDCl₃): δ 170.7, 169.7, 169.4, 132.3, 132.1, 129.4, 128.1, 85.1, 71.9, 68.6, 68.5, 62.1, 62.0, 20.9, 20.8 (2) ppm. HRMS: Calcd for C₁₈H₂₁O₇N₃NaS [M+Na]+: 446.0992; found 446.0987 (-1,6 ppm). M.p.: 125-127 °C. [α]_{D}²⁰: +167,5 (c 1, CHCl₃).

### General procedure for the synthesis of natural open-chain isothiocyanates

To a solution of 3 (100 mol%) in Et₂O (0.07 M), PPh₃ (250 mol%) is added at room temperature and stirred under reflux. After the starting product is consumed (monitoring by TLC), the solvent is evaporated under reduced pressure, the reaction crude is dissolved in CS₂ (0.12 M) and stirring under reflux for 3 hours. Then, the excess of CS₂ is evaporated under reduced pressure and the crude obtained is purified by flash column chromatography.

### (3-isothiocyanatopropyl)(methyl)sulfane, Iberverin 4

The reaction is carried out following the general procedure starting from **3** (0.94 g, 7.17 mmol), Et₂O (96 mL), PPh₃ (4.70 g, 17.93 mmol) and CS₂ (38 mL). The crude is purified by flash column chromatography (hexane) to obtain 0.77 g, 5.24 mmol (73% yield) of the isothiocyanate **4** as a yellow liquid. ¹H-NMR (500 MHz, CDCl₃): δ 3.67 (t, *J* = 6.4 Hz, 2H, - OCH₂-), 2.61 (t, *J* = 6.9 Hz, 2H, -CH₂NCS), 2.11 (s, 3H, -SCH₃), 1.97 (quint, *J* = 6.7 Hz, 2H, -CH₂CH₂CH₂-) ppm. ¹³C-NMR (125 MHz, CDCl₃): δ 130.9 (-NCS), 43.8, 31.0, 29.3, 15.7 ppm. HRMS: Calcd for C₅H₁₀NS₂ [M+H]+: 148.0249; found: 148.0246 (-2.1 ppm).

### General procedure for the synthesis of glycomimetic isothiocyanates

a) Method A: To a solution of the corresponding azide (100 mol%) in Et₂O, PPh₃ (250 mol%) is added at room temperature and it is kept stirring under reflux. Once the starting product is consumed (monitoring by TLC), the solvent is evaporated under reduced pressure. The crude is dissolved in CS₂ and stirring at reflux until the no evolution of the reaction (monitoring by TLC). Then, the excess of CS₂ is evaporated under reduced pressure and the crude is purified by flash column chromatography.
b) Method B: To a solution of the corresponding azide (100 mol%) in Et₂O, PPh₃ (250 mol%) is added at room temperature and kept stirring under reflux. After the starting product is consumed (monitoring by TLC), the solvent is evaporated under reduced pressure. The mixture is dissolved in CS₂ and heated in the microwave at 150 ºC for 15 minutes. The crude is purified by flash column chromatography.

### Methyl 2,4,6-tri-O-acetyl-3-isothiocyanato-1,3-dideoxy-1-thio-β-D-glucopyranoside, 13β

a) Method A: The reaction is carried out following the general procedure starting from **10β** (0.275 g, 0.76 mmol), Et₂O (8.8 mL), PPh₃ (0.499 g, 1.90 mmol) and CS₂ (3.7mL). The crude is purified by flash column chromatography (AcOEt/hexane 1:5) to obtain 0.078 g, 0.21 mmol (27 % yield) of the isothiocyanate **13β** as a beige syrup.
b) Method B: The reaction is carried out following the general procedure starting from **10β** (0.14 g, 0.40 mmol), Et₂O (5.0 mL), PPh₃ (0.26 g, 0.99 mmol) and CS₂ (5.0 mL). The crude is purified by flash column chromatography (AcOEt/hexane 1:5) to obtain 0.107 g, 0.28 mmol (72% yield) of the isothiocyanate **13β** as a beige syrup. ¹H-NMR (500 MHz, CDCl₃): δ 5.08 (t, *J* = 10.1 Hz, 1H, H₄), 5.05 (t, *J* = 9.8 Hz, 1H, H₂), 4.29 (d, *J* = 9.8 Hz, 1H, H₁), 4.21 (dd, *J* = 4.8 and 12.4 Hz, 1H, H₆), 4.11 (dd, *J* = 2.5 and 12.5 Hz, 1H, H₆), 4.04 (t, *J* = 10.1 Hz, 1H, H₃), 3.63 (ddd, *J* = 2.5, 4.8, and 9.9 Hz, 1H, H₅), 2.15 (s, 3H, CH₃COO-), 2.14 (s, 3H, CH₃COO-), 2.12 (s, 3H, CH₃COO-), 2.07 (s, 3H, -SCH₃) ppm.¹³C-NMR (125 MHz, CDCl₃): δ 170.6, 169.0 (2), 140.9 (-NCS), 82.9, 76.4, 69.0, 68.2, 62.4, 62.0, 20.8, 20.7 (2), 11.1 ppm. HRMS: Calcd for C₁₄H₁₉O₇NNaS₂ [M+Na]+: 400.0495; found 400.0492 (-0.8 ppm).

### Ethyl 2,4,6-tri-O-acetyl-3-isothiocyanato-1,3-dideoxy-1-thio-β-D-glucopyranoside, 14β

a) Method A: The reaction is carried out following the general procedure starting from **11β** (0.317 g, 0.84 mmol), Et₂O (10.0 mL), PPh₃ (0.553 g, 2.11 mmol) and CS₂ (10.0 mL). The crude is purified by flash column chromatography (AcOEt/hexane 1:5) to obtain 0.074 g, 0.19 mmol (23 % yield) of the isothiocyanate **14β** as a beige syrup.
b) Method B: The reaction is carried out following the general procedure starting from **11β** (0.086 g, 0.36 mmol), Et₂O (5.0 mL), PPh₃ (0.150 g, 0.57 mmol) and CS₂ (5.0 mL). The crude is purified by flash column chromatography (AcOEt/hexane 1:5) to obtain 0.058 g, 0.15 mmol (64% yield) of the isothiocyanate **14β** as a beige syrup. ¹H-NMR (500 MHz, CDCl₃): δ 5.65 (d, *J* = 5.6 Hz, 1H, H₁), 5.10 (t, *J* = 10.0 Hz, 1H, H₄), 5.00 (dd, *J* = 5.6 and 10.7 Hz, 1H, H₂), 4.32 (ddd, *J* = 2.3, 4.7 and 10.0 Hz, 1H, H₅), 4.27 - 4.22 (m, 2H, H₆ and H₃), 4,06 (dd, *J* = 2.3 and 12.3 Hz, 1H, H_{6'}), 2.62 - 2.49 (m, 2H, -CH₂CH₃), 2.15 (s, 3H, CH₃COO-), 2.14 (s, 3H, CH₃COO-), 2.08 (s, 3H, CH₃COO-), 1.27 (t, *J* = 7.4 Hz, 3H, -CH₂CH₃) ppm. ¹³C-NMR (125 MHz, CDCl₃): δ 170.7, 169.7, 169.3, 141.1 (-NCS), 81.6, 71.2, 68.5, 67.8, 61.9, 58.9, 24.4, 20.9, 20.8 (2), 14.8 ppm. HRMS: Calcd for C₁₅H₂₁O₇NNaS₂ [M+Na]+: 414.0652; found 414.0649 (-0.7 ppm).

### Phenyl 2,4,6-tri-O-acetyl-3-isothiocyanato-1,3-dideoxy-1-thio-β-D-glucopyranoside, 15β

a) Method A: The reaction is carried out following the general procedure starting from **12β** (0.80 g, 1.89 mmol), Et₂O (22.0 mL), PPh₃ (1.24 g, 4.72 mmol) and CS₂ (10.0 mL). The crude is purified by flash column chromatography (AcOEt/hexane 1:5) to obtain 0.163 g, 0.37 mmol (17 % yield) of the isothiocyanate **15β** as a brown solid.
b) Method B: The reaction is carried out following the general procedure starting from **12β** (0.150 g, 0.35 mmol), Et₂O (5.0 mL), PPh₃ (0.232 g, 0.88 mmol) and CS₂ (5.0 mL). The crude is purified by flash column chromatography (AcOEt/hexane 1:5) to obtain 0.118 g, 0.27 mmol (76% yield) of the isothiocyanate **15β** as a brown solid. ¹H-NMR (500 MHz, CDCl₃): δ 7.51 - 7.47 (m, 2H, SC₆H₅), 7.35 - 7.30 (m, 3H, SC₆H₅), 5.05 (t, *J* = 10.0 Hz, 1H, H₂), 4.98 (t, *J* = 9.9 Hz, 1H, H₄), 4.62 (d, *J* = 9.9 Hz, 1H, H₁), 4.21- 4.15 (m, 2H, H₆, H_{6'}), 4.06 (t, *J* = 10.0 Hz, 1H, H₃), 3.64 (ddd, *J* = 3.0, 4.8 and 9.9 Hz, 1H, H₅), 2.19 (s, 3H, CH₃COO-), 2.13 (s, 3H, CH₃COO-), 2.08 (s, 3H, CH₃COO-) ppm. ¹³C-NMR (125 MHz, CDCl₃): δ 170.7, 169.1, 169.0, 141.1 (-NCS), 133.3, 131.6, 129.1, 128.7, 86.2, 76.4, 70.0, 68.4, 62.6, 62.2, 21.0, 20.9, 20.8 ppm. HRMS: Calcd for C₁₉H₂₁O₇NNaS₂ [M+Na]+: 462.0652; found 462.0642 (-2.1 ppm).

### Ethyl 2,4,6-tri-O-acetyl-3-isothiocyanato-1,3-dideoxy-1-thio-α-D-glucopyranoside, 14α

a) Method A: The reaction is carried out following the general procedure starting from **11α** (0.304 g, 0.81 mmol), Et₂O (10.0 mL), PPh₃ (0.532 g, 2.03 mmol) and CS₂ (5.0 mL). The crude is purified by flash column chromatography (AcOEt/hexane 1:10) to obtain 0.087 g, 0.22 mmol (28 % yield) of the isothiocyanate **14α** as a beige syrup.
b) Method B: The reaction is carried out following the general procedure starting from **11α** (0.100 g, 0.27 mmol), Et₂O (5.0 mL), PPh₃ (0.174 g, 0.67 mmol) and CS₂ (5.0 mL). The crude is purified by flash column chromatography (AcOEt/hexane 1:7) to obtain 0.077 g, 0.20 mmol (73% yield) of the isothiocyanate **14α** as a beige syrup. ¹H-NMR (500 MHz, CDCl₃): δ 5.65 (d, *J* = 5.6 Hz, 1H, H₁), 5.10 (t, *J* = 10.0 Hz, 1H, H₄), 5.00 (dd, *J* = 5.6 and 10.7 Hz, 1H, H₂), 4.32 (ddd, *J* = 2.3, 4.7 and 10.0 Hz, 1H, H₅), 4.27 - 4.22 (m, 2H, H₆ and H₃), 4.06 (dd, *J* = 2.3 and 12.3 Hz, 1H, H_{6'}), 2.62 - 2.49 (m, 2H, -CH₂CH₃), 2.15 (s, 3H, CH₃COO-), 2.14 (s, 3H, CH₃COO-), 2.08 (s, 3H, CH₃COO-), 1.27 (t, *J* = 7.4 Hz, 3H, -CH₂CH₃) ppm. ¹³C-NMR (125 MHz, CDCl₃): δ 170.7, 169.7, 169.3, 141.1 (-NCS), 81.6, 71.2, 68.5, 67.8, 61.9, 58.9, 24.4, 20.9, 20.8 (2), 14.8 ppm. HRMS: Calcd for C₁₅H₂₁O₇NNaS₂ [M+Na]+: 414.0652; found 414.0649 (-0,7 ppm).

### Phenyl 2,4,6-tri-O-acetyl-3-isothiocyanato-1,3-dideoxy-1-thio-α-D-glucopyranoside, 15α

a) Method A: The reaction is carried out following the general procedure starting from **12α** (0.182 g, 0.43 mmol), Et₂O (8.0 mL), PPh₃ (0.281 g, 1.07 mmol) and CS₂ (4.0 mL). The crude is purified by flash column chromatography (AcOEt/hexane 1:10) to obtain 0.035 g, 0.08 mmol (19 % yield) of the isothiocyanate **15α** as a beige syrup.
b) Method B: The reaction is carried out following the general procedure starting from **12α** (0.206 g, 0.49 mmol), Et₂O (6.0 mL), PPh₃ (0.319 g, 1.22 mmol) and CS₂ (5.0 mL). The crude is purified by flash column chromatography (AcOEt/hexane 1:7) to obtain 0.130 g, 0.30 mmol (61 % yield) of the isothiocyanate **15α** as a beige syrup. ¹H-NMR (500 MHz, CDCl₃): δ 7.45 - 7.40 (m, 2H, SC₆H₅), 7.33 - 7.29 (m, 3H, SC₆H₅), 5.87 (d, *J* = 5.5 Hz, 1H, H₁), 5.12 (t, *J* = 10.0 Hz, 1H, H₄), 5.08 (dd, *J* = 5.5 and 10.8 Hz, 1H, H₂), 4.45 (ddd, *J* = 2.3, 5.2 and 10.1 Hz, 1H, H₅), 4.32 (t, *J* = 10.4 Hz, 1H, H₃), 4.23 (dd, *J* = 5.2 and 12.4 Hz, 1H, H₆), 4.02 (dd, *J* = 2.3 and 12.4 Hz, 1H, H_{6'}), 2.19 (s, 3H, CH₃COO-), 2.17 (s, 3H, CH₃COO-), 2.02 (s, 3H, CH₃COO-) ppm. ¹³C-NMR (125 MHz, CDCl₃): δ 170.7, 169.7, 169.3, 141.5 (-NCS), 132.0, 129.4, 128.2, 84.9, 71.5, 68.5, 68.4, 61.9, 59.0, 20.9 (2), 20.8 ppm. HRMS: Calcd for C₁₉H₂₁O₇NNaS₂ [M+Na]+: 462.0652; found 462.0648 (-0.8 ppm).

### General procedure for the oxidation of thioether to sulfoxide.

To a solution of the corresponding thioderivative (100 mol%) in CH₂Cl₂ (0.5 M) under argon atmosphere, a solution of m-CPBA (110 mmol%) in CHCl₃ (0.18 M) is added at -78 °C. After checking by TLC that the starting product is completely consumed, the reaction is extracted with saturated NaHCO₃ solution (3 × 40 mL), washed with saturated NaCl solution (30 mL), dried over anhydrous Na₂SO₄, filtered and the solvent is evaporated under reduced pressure. The crude is purified by flash column chromatography using the eluent indicated in each case.

### 1-isothiocyanato-3-(methylsulfinyl)propane, rac-lberin rac-5

The reaction is carried out following the general procedure starting from **4** (0.30 g, 2.04 mmol) in CH₂Cl₂ (2 mL) and *m*-CPBA (0.53 g, 2, 24 mmol) in 5.5 mL of CHCl₃. After 10 minutes the starting product is completely consumed. The crude is purified by flash column chromatography (CH₂Cl₂/MeOH 40:1) to obtain 0.28 g, 1.72 mmol (83% yield) of ***rac*-lberin, rac-5,** as a yellow liquid. ¹H-NMR (500 MHz, CDCl₃): δ 3.80 - 3.70 (m, 2H, - OCH₂-), 2.85 - 2.75 (m, 2H, -CH₂NCS), 2.63 (s, 3H, -SCH₃), 2.25 - 2.19 (m, 2H, - CH₂CH₂CH₂-) ppm. ¹³C-NMR (125 MHz, CDCl₃): δ 132.4 (-NCS), 51.2, 44.3, 39.1, 23.7 ppm. HRMS: Calcd for C₅H₉NNaS₂ [M+Na]+: 186.0018; found 186.0016 (-2,1 ppm).

### Methyl 2,4,6-tri-O-acetyl-3-isothiocyanato-1,3-dideoxy-1-(S)-sulfinyl-β-D-glucopyranoside, (Sₛ)-16β

The reaction is carried out following the general procedure starting from **13β** (0.020 g, 0.05 mmol) in CH₂Cl₂ (1.5 mL) and *m*-CPBA (0.014 g, 0.06 mmol) in 2 mL of CHCl₃. After 10 minutes, the starting product is completely consumed. The crude is purified by flash column chromatography (CH₂Cl₂/MeOH 30:1) to obtain 0.018 g, 0.04 mmol (86 % yield) of sulfoxide (S)-**16β** as a yellow syrup. ¹H-NMR (500 MHz, CDCl₃): δ 5.13 (t, *J* = 9.9 Hz, 1H, H₂), 5.01 (t, *J* = 10.0 Hz, 1H, H₄), 4.31 - 4.28 (m, 2H, H₁ and H₆), 4.20 - 4.14 (m, 2H, H₃ and H_{6'}), 3.76 - 3.74 (m, 1H, H₅), 2.68 (s, 3H, -SCH₃), 2.16 (s, 3H, CH₃COO-), 2.14 (s, 3H, CH₃COO-), 2.08 (s, 3H, CH₃COO-) ppm. ¹³C-NMR (125 MHz, CDCl₃): δ 170.6, 169.6, 169.1, 142.5 (-NCS), 91.1, 77.2, 68.8, 67.7, 62.0, 61.4, 32.7, 20.8 (2), 20.7 ppm. HRMS: Calcd for C₁₄H₁₉O₈NNaS₂ [M+Na]+: 416.0444; found 416.0441 (-0,8 ppm). [α]_{D}²⁰: -15.0 (c 1, CHCl₃).

### Ethyl 2,4,6-tri-O-acetyl-3-isotiocianato-1,3-dideoxy-1[(S and R)-sulfinyl]-β-D-glucopyranose

The reaction is carried out following the general procedure starting from **14β** (0.062 g, 0.16 mmol) in CH₂Cl₂ (1.5 mL) and *m*-CPBA (0.041 g, 0.17 mmol) in 2 mL of CHCl₃. After 10 minutes, the starting product is completely consumed. The crude is purified by flash column chromatography (CH₂Cl₂/MeOH 30:1) to obtain 0.018 g, 0.04 mmol (63 % yield) of sulfoxide (*S*)-**17β** as a yellow solid and 0.009 g 0.02 mmol (14 % yield) of the sulfoxide (*R*)-**17β** as a white solid.

### Ethyl 2,4,6-tri-O-acetyl-3-isotiocianato-1,3-dideoxy-1[(S)-sulfinyl]-β-D-glucopyranose, (Sₛ)-17β

¹H-NMR (500 MHz, CDCl₃): δ 5.20 (t, *J* = 10.0 Hz, 1H, H₂), 5.11 (t, *J* = 10.0 Hz, 1H, H₄), 4.26 (dd, *J* = 4.7 and 12.7 Hz, 1H, H₆), 4.22 (d, *J* = 10.2 Hz, 1H, H₁), 4.17 (dd, *J* = 2.4 and 12.7 Hz, 1H, H_{6'}), 4.15 (t, *J* = 10.0 Hz, 1H, H₃), 3.70 (ddd, *J* = 2.4, 4.6 and 9.9 Hz, 1H, H₅), 2.96 - 2.84 (m, 2H, -CH₂CH₃), 2.17 (s, 3H, CH₃COO- ), 2.15 (s, 3H, CH₃COO-), 2.09 (s, 3H, CH₃COO-), 1.38 (t, *J* = 7,5 Hz, 3H, -CH₂CH₃) ppm. ¹³C-NMR (125 MHz, CDCl₃): δ 170.7, 169.6, 169.1, 142.4 (-NCS), 90.5, 77.5, 69.0, 67.8, 62.1, 61.5, 41.3, 20.8 (2), 6.6 ppm. HRMS: Calcd for C₁₅H₂₁O₈NNaS₂ [M+Na]+: 430.0601; found 430.0600 (-0,3 ppm). D.r.: 80:20 (SS:RS). [α]_{D}²⁰: +44.3 (c 1, CHCl₃).

### Ethyl 2,4,6-tri-O-acetyl-3-isotiocianato-1,3-dideoxy-1[(R)-sulfinyl]-β-D-glucopyranose, (Rₛ)-17β

¹H-NMR (500 MHz, CDCl₃): δ 5.50 (t, *J* = 10.0 Hz, 1H, H₂), 5.13 (t, *J* = 10.0 Hz, 1H, H₄), 4.27 - 4.18 (m, 3H, H₃, H₆ and H_{6'}), 4.12 (d, *J* = 9.9 Hz, 1H, H₁), 3.73 (ddd, *J* = 3.1, 4.8 and 9.9 Hz, 1H, H₅), 3.17 - 3.04 (m, 1H, -CH₂CH₃), 2.81 - 2.74 (m, 1H, -CH₂CH₃), 2.17 (s, 3H, CH₃COO-), 2.15 (s, 3H, CH₃COO-), 2.08 (s, 3H, CH₃COO-), 1.34 (t, *J* = 7.6 Hz, 3H, -CH₂CH₃) ppm. ¹³C-RMN (125 MHz, CDCl₃): δ 170.7, 169.0, 168.6, 141.8 (-NCS), 86.7, 77.4, 68.2, 67.0, 62.3, 62.0, 41.4, 20.8 (2), 7.5 ppm. HRMS: Calcd for C₁₅H₂₁O₈NNaS₂ [M+Na]+: 430.0601; found 430.0604 (0,7 ppm). D.r.: 80:20 (Ss:Rs).

### Phenyl 2,4,6-tri-O-acetyl-3-isotiocianato-1,3-dideoxy-1[(S and R)-sulfinyl]-β-D-glucopyranose

The reaction is carried out following the general procedure starting from **15β** (0.221 g, 0.50 mmol) in CH₂Cl₂ (1.5 mL) and *m*-CPBA (0.130 g, 0.55 mmol) in 2 mL of CHCl₃. After 8 hours, the starting product is completely consumed. The crude is purified by flash column chromatography (Hexane/ CH₂Cl₂/*tert-*butyl methyl ether 1:1:1) to obtain 0.109 g, 0.24 mmol (48 % yield) of sulfoxide (*S*)-**18β** as a yellow solid and 0.032 g, 0.07 mmol (14 % yield) of the sulfoxide (*R*)-**18β** as a white solid.

### Phenyl 2,4,6-tri-O-acetyl-3-isotiocianato-1,3-dideoxy-1[(S)-sulfinyl]-β-D-glucopyranose, (Sₛ)-18β

¹H-NMR (500 MHz, CDCl₃): δ 7.71 - 7.70 (m, 2H, SC₆H₅), 7.56 - 7.51 (m, 3H, SC₆H₅), 5.23 (t, *J* = 9.9 Hz, 1H, H₂), 4.94 (t, *J* = 10.0 Hz, 1H, H₄), 4.27 (d, *J* = 9.9 Hz, 1H, H₁), 4.13 - 4.03 (m, 3H, H₃, H₆ and H_{6'}), 3.57 (ddd, *J* = 2.6,4.6 and 9.9 Hz, 1H, H₅), 2.10 (s, 3H, CH₃COO-), 2.09 (s, 3H, CH₃COO-), 1.97 (s, 3H, CH₃COO- ) ppm. ¹³C-NMR (125 MHz, CDCl₃): δ 170.4, 169.3, 169.0, 141.9 (-NCS), 138.9, 132.0, 129.0, 126.2, 92.4, 77.2, 68.2, 67.7, 62.2, 61.3, 20.9, 20.7 (2), ppm. HRMS: Calcd for C₁₉H₂₁O₈NNaS₂ [M+Na]+: 478.0601; found 478.0594 (-1.3 ppm). [α]_{D}²⁰: +9.2 (c 1, CHCl₃).

### Phenyl 2,4,6-tri-O-acetyl-3-isotiocianato-1,3-dideoxy-1[(R)-sulfinyl]-β-D-glucopyranose, (Rₛ)-18β

¹H-NMR (500 MHz, CDCl₃): δ 7.65 - 7.63 (m, 2H, SC₆H₅), 7.57 - 7.51 (m, 3H, SC₆H₅), 5.28 (t, *J* = 9.8 Hz, 1H, H₂), 4.98 (t, *J* = 10.0 Hz, 1H, H₄), 4.23 (d, *J* = 9.8 Hz, 1H, H₁), 4.14 (t, *J* = 10,0 Hz, 1H, H₃), 4.10 (dd, *J* = 5.5 y 12.4 Hz, 1H, H₆), 4.04 (dd, *J* = 2.5 y 12.4 Hz, 1H, H_{6'}), 3.55 (ddd, *J* = 2.5, 5.6 y 9.8 Hz 1H, H₅), 2.18 (s, 3H, CH₃COO-), 2.11 (s, 3H, CH₃COO-), 1.96 (s, 3H, CH₃COO-) ppm. ¹³C-NMR (125 MHz, CDCl₃): δ 170.5, 169.0, 168.7, 141.7 (-NCS), 138.6, 132.0, 129.1, 126.0, 89.7, 68.1, 67.5, 62, 3, 61.7, 62.2, 20.9, 20.8, ppm. [α]_{D}²⁰: -120,1 (*c* 1, CHCl₃).

### Ethyl 2,4,6-tri-O-acetyl-3-isothiocyanato-1,3-dideoxy-1[(R)-sulfinyl]-α-D-glucopyranse, (Rₛ)-17α

The reaction is carried out following the general procedure starting from **14α** (0.100 g, 0.26 mmol) in CH₂Cl₂ (1.5 mL) and *m*-CPBA (0.066 g, 0.28 mmol) in 2 mL of CHCl₃. After 8 hours, the starting product is completely consumed. The crude is purified by flash column chromatography (Hexane/AcOEt 1:3) to obtain 0.068 g, 0.17 mmol (65 % yield) of sulfoxide (*R*)**-17α** as a yellow liquid. ¹H-NMR (500 MHz, CDCl₃): δ 5.31 (dd, *J* = 5.6 and 10.3 Hz, 1H, H₂), 5.11 (t, *J* = 9.6 Hz, 1H, H₄), 4.81 (d, *J* = 5.5 Hz, 1H, H₁), 4.71 (t, *J* = 9.7 Hz, 1H, H₃), 4.15 (dd, *J* = 5.3 and 12.5 Hz, 1H, H₆), 4.09 (dd, *J* = 2.5 and 12.5 Hz, 1H, H_{6'}), 3.86 (ddd, *J* = 2.5, 5.4 and 9.8 Hz, 1H, H₅), 2.95 - 2.79 (m, 2H, -CH₂CH₃), 2.18 (s, 3H, CH₃COO-), 2.15 (s, 3H, CH₃COO-), 2.09 (s, 3H, CH₃COO-), 1.40 (t, *J* = 7.4 Hz, 3H, -CH₂CH₃) ppm. ¹³C-NMR (125 MHz, CDCl₃): δ 170.3, 170.1, 169.2, 142.3 (-NCS), 87.3, 73.9, 70.7, 68.2, 62.0, 58.5, 43.0, 20.8 (3), 6.0 ppm. HRMS: Calcd for C₁₅H₂₁O₈NNaS₂ [M+Na]+: 430.0601; found 430.0598 (-0.7 ppm). [α]_{D}²⁰: +87.1 (c 1, CHCl₃).

### Phenyl 2,4,6-tri-O-acetyl-3-isothiocyanato-1,3-dideoxy-1[(R)-sulfinyl]-α-D-glucopyranose, (Rₛ)-18α

The reaction is carried out following the general procedure starting from **15α** (0.038 g, 0.09 mmol) in CH₂Cl₂ (1.5 mL) and *m*-CPBA (0.022 g, 0.09 mmol) in 2 mL of CHCl₃. After 8 hours, the starting product is completely consumed. The crude is purified by flash column chromatography (Hexane/AcOEt 1:3) to obtain 0.021 g, 0.05 mmol (54 % yield) of sulfoxide (*R*)**-18α** as a yellow solid. ¹H-NMR (500 MHz, CDCl₃): δ 7.64 - 7.62 (m, 2H, SC₆H₅), 7.58 - 7.51 (m, 3H, SC₆H₅), 5.26 - 5.22 (m, 2H, H₂ and H₄), 5.19 - 5.10 (m, 1H, H₃), 4.94 (d, *J* = 4.6 Hz, 1H, H₁), 4.49 (ddd, *J* = 2.3, 4.3 and 9.9 Hz, 1H, H₅), 4.21 (dd, *J* = 4.5 and 12.7 Hz, 1H, H₆), 4.09 (dd, *J* = 2.3 y 12.6 Hz, 1H, H_{6'}), 2.15 (s, 3H, CH₃COO-), 2.08 (s, 3H, CH₃COO-), 1.68 (s, 3H, CH₃COO-) ppm. ¹³C-NMR (125 MHz, CDCl₃): δ 170.6, 167.0, 169.2, 141.5, 140.3, 131.4, 129.6, 124.8, 91.8, 73.8, 71.6, 68.2, 61.7, 57.7, 20.8, 20.2 ppm. HRMS: Calcd for C₁₉H₂₁O₈NNaS₂ [M+Na]+: 478.0601; found 478.0594 (-1.4 ppm). [α]_{D}²⁰: -41.1 (c 1, CHCl₃).

### General procedure for the oxidation of thioether to sulfone.

To a solution of the corresponding thioderivative (100 mol%) in CH₂Cl₂ (0.6 M) under argon atmosphere, a solution of m-CPBA (210-250 mol%) in CHCl₃ (0.2M) is added at room temperature. After checking by TLC that the starting product is completely consumed, the reaction is extracted with saturated NaHCO₃ solution (3 × 20 mL), washed with saturated NaCl solution (20 mL), dried over anhydrous Na₂SO₄, filtered and the solvent is evaporated under reduced pressure. The crude is purified by flash column chromatography using the eluent indicated in each case.

### 1-isothiocyanato-3-(methylsulfonyl)propane, Cheirolin 6

The reaction is carried out following the general procedure starting from Iberverin, **4**, (0.15 g, 1.02 mmol) in 4 mL of CH₂Cl₂ and m-CPBA (0.51 g, 2.14 mmol) in 12 mL of CHCl₃. The crude is purified by flash column chromatography (AcOEt/hexane 1:1) to obtain 0.114 g, 1.51 mmol (62 % yield) of the sulfone Cheirolin, **6**, as a beige liquid. ¹H-NMR (500 MHz, CDCl₃): δ 3.78 (t, *J* = 6.3 Hz, 2H, - OCH₂-), 3.16 (t, *J* = 7.5 Hz, 2H, -CH₂NCS), 2.97 (s, 3H, -SCH₃), 2.28 - 2.23 (m, 2H, - CH₂CH₂CH₂-) ppm. ¹³C-NMR (125 MHz, CDCl₃): δ 133.1 (-NCS), 51.6, 43.8, 41.4, 23.1 ppm. HRMS: Calcd for C₅H₉NNaS₂ [M+Na]+: 186.0018; found 186.0016 (-2.1 ppm).

### Methyl 2,4,6-tri-O-acetyl-3-isothiocyanato-1,3-dideoxy-1-sulfonyl-β-D-glucopyranose, 19β

The reaction is carried out following the general procedure starting from **13β** (0.020 g, 0.05 mmol) in 1,5 mL of CH₂Cl₂ and *m*-CPBA (0.026 g, 0.11 mmol) in 2 mL of CHCl₃. The crude is purified by flash column chromatography (*tert*-butyl methyl ether/hexane 3:1) to obtain 0.013 g, 0.03 mmol (60 % yield) of the sulfone **19β** as a white solid. ¹H-NMR (500 MHz, CDCl₃): δ 5.38 (t, *J* = 9.9 Hz, 1H, H₂), 5.15 (t, *J* = 10.0 Hz, 1H, H₄), 4.29 (dd, *J* = 4.8 and 12.8 Hz, 1H, H₆), 2.24 (d, *J* = 9.9 Hz, 1H, H₁), 4.20 - 4.14 (m, 2H, H₃ and H_{6'}), 3.76 (ddd, *J* = 2.3, 4.8 and 9.9 Hz, 1H, H₅), 2.96 (s, 3H, -SCH₃), 2.16 (s, 3H, CH₃COO-), 2.15 (s, 3H, CH₃COO-), 2.09 (s, 3H, CH₃COO-) ppm. ¹³C-NMR (125 MHz, CDCl₃): δ 170.6, 169.5, 169.0, 142.6 (-NCS), 88.7, 77.2, 67.6, 67.0, 62.0, 61.4, 36.4, 20.8 (3) ppm. HRMS: Calcd for C₁₄H₁₉O₉NNaS₂ [M+Na]+: 432.0387; found 432.0387 (-1,6 ppm).

### Ethyl 2,4,6-tri-O-acetyl-3-isothiocyanato-1,3-dideoxy-1-sulfonyl-β-D-glucopyranose, 20β

The reaction is carried out following the general procedure starting from **14β** (0.050 g, 0.13 mmol) in 1,5 mL of CH₂Cl₂ and *m*-CPBA (0.063 g, 0.27 mmol) in 2 mL of CHCl₃. The crude is purified by flash column chromatography (tert-butyl methyl ether/hexane 3:1) to obtain 0.033 g, 0.08 mmol (60 % yield) of the sulfone **20β** as a white solid. 1H-NMR: (500 MHz, CDCl₃): δ 5.44 (t, *J* = 9.9 Hz, 1H, H₂), 5.13 (t, *J* = 10.0 Hz, 1H, H₄), 4.34 (d, *J* = 9.9 Hz, 1H, H₁), 4.25 (dd, *J* = 4.9 and 12.7 Hz, 1H, H₆), 4.20 - 4.15 (m, 2H, H₃ and H_{6'}), 3.73 (ddd, *J* = 2.4, 4.8 and 9.9 Hz, 1H, H₅), 3.22 - 3.08 (m, 2H, -CH₂CH₃), 2.16 (s, 3H, CH₃COO-), 2.15 (s, 3H, CH₃COO-), 2.09 (s, 3H, CH₃COO-), 1.40 (t, *J* = 7.5 Hz, 3H, -CH₂CH₃) ppm. ¹³C-NMR (125 MHz, CDCl₃): δ 170.6, 169.2, 169.0, 142.5 (-NCS), 88.0, 67.6, 66.7, 62.0, 61.4, 43.9, 28.7, 20.8 (3), 5.4 ppm. HRMS: Calcd for C₁₅H₂₁O₉NNaS₂ [M+Na]+: 446.0550; found 4346.0545 (-1.2 ppm).

### Phenyl 2,4,6-tri-O-acetyl-3-isothiocyanato-1,3-dideoxy-1-sulfonyl-β-D-glucopyranose, 21β

The reaction is carried out following the general procedure starting from **15β** (0.020 g, 0.05 mmol) in 2 mL of CH₂Cl₂ and *m*-CPBA (0.028 g, 0.12 mmol) in 3 mL of CHCl₃. The crude is purified by flash column chromatography (AcOEt/hexane 1:2) to obtain 0.014 g, 0.03 mmol (63 % yield) of the sulfone **21β** as a white solid. ¹H-NMR (500 MHz, CDCl₃): δ 7.92 - 7.91 (m, 2H, SC₆H₅), 7.75 - 7.71 (m, 1H, SC₆H₅), 7.61 - 7.58 (m, 2H, SC₆H₅), 5.17 (t, *J* = 9.8 Hz, 1H, H₂), 4.88 (t, *J* = 10.0 Hz, 1H, H₄), 4.42 (d, *J* = 9.8 Hz, 1H, H₁), 4.12 - 4.08 (m, 3H, H₃, H₆ and H_{6'}), 3.65 - 3.62 (m, 1H, H₅), 2.23 (s, 3H, CH₃COO-), 2.11 (s, 3H, CH₃COO-), 1.98 (s, 3H, CH₃COO-) ppm. ¹³C-NMR (125 MHz, CDCl₃): δ 170.3, 169.1, 169.0, 142.1 (-NCS), 134.9, 134.5, 130.8, 129.1, 89.1, 76.8, 67.5, 67.0, 62.2, 61.2, 20.9, 20.7 (2), ppm. HRMS: Calcd for C₁₉H₂₁O₉NNaS₂ [M+Na]+: 494.0550; found 494.0550 (0.1 ppm).

### Ethyl 2,4,6-tri-O-acetyl-3-isothiocyanato-1,3-dideoxy-1-sulfonyl-α-D-glucopyranose, 20α

The reaction is carried out following the general procedure starting from **14α** (0.020 g, 0.05 mmol) in 2 mL of CH₂Cl₂ and *m*-CPBA (0.031 g, 0.13 mmol) in 3 mL of CHCl₃. The crude is purified by flash column chromatography (*tert*-butyl methyl ether/hexane 1:1) to obtain 0.016 g, 0.04 mmol (73 % yield) of the sulfone **20α** as a white solid. ¹H-NMR (500 MHz, CDCl₃): δ 5.23 (d, *J* = 6.7 Hz, 1H, H₁), 5.18 (dd, *J* = 6.6 and 10.5 Hz, 1H, H₂), 5.11 (t, *J* = 10.1 Hz, 1H, H₄), 4.99 (t, *J* = 10.3 Hz, 1H, H₃), 4.53 (ddd, *J* = 2.4, 5.1 and 10.0 Hz, 1H, H₅), 4.19 (dd, *J* = 5.1 and 12.6 Hz, 1H, H₆), 4.11 (dd, *J* = 2.4 and 12.6 Hz, 1H, H_{6'}), 3.18 - 2.99 (m, 2H, -CH₂CH₃), 2.20 (s, 3H, CH₃COO-), 2.16 (s, 3H, CH₃COO-), 2.08 (s, 3H, CH₃COO-), 1.39 (t, *J* = 7.4 Hz, 3H, -CH₂CH₃) ppm. ¹³C-NMR (125 MHz, CDCl₃): δ 170.6, 170.4, 169.3, 142.4 (-NCS), 82.7, 72.3, 69.6, 67.6, 62.0, 57.5, 46.4, 20.8, 20.7, 6.2 ppm. HRMS: Calcd for C₁₅H₂₁O₉NNaS₂ [M+Na]+: 446.0550; found 446.0545 (-1.0 ppm).

### Phenyl 2,4,6-tri-O-acetyl-3-isothiocyanato-1,3-dideoxy-1-sulfonyl-α-D-glucopyranose, 21α

The reaction is carried out following the general procedure starting from **15α** (0.047 g, 0.12 mmol) in 1.5 mL of CH₂Cl₂ and m-CPBA (0.053 g, 0.22 mmol) in 2 mL of CHCl₃. The crude is purified by flash column chromatography (tert-butyl methyl ether/hexane 1:2) to obtain 0.029 g, 0.06 mmol (58 % yield) of the sulfone **21α** as a beige syrup. ¹H-NMR (500 MHz, CDCl₃): δ 7.89 - 7.88 (m, 2H, SC₆H₅), 7.74 - 7.70 (m, 1H, SC₆H₅), 7.62 - 7.59 (m, 2H, SC₆H₅), 5.23 - 5.18 (m, 2H, H₂ and H₄), 5.14 - 5.09 (m, 2H, H₁ and H₃), 4.56 (ddd, *J* = 2.3, 4.4 and 9.9 Hz, 1H, H₅), 4.21 (dd, *J* = 4.6 and 12.7 Hz, 1H, H₆), 3.79 (dd, *J* = 2.4 and 12.7 Hz, 1H, H_{6'}), 2.20 (s, 3H, CH₃COO-), 2.17 (s, 3H, CH₃COO-), 1.98 (s, 3H, CH₃COO-) ppm. ¹³C-NMR (125 MHz, CDCl₃): δ 170.6, 170.5, 169.2, 142.3 (-N*C*S), 137.4, 134.8, 129.6, 128.9, 85.5, 72.1, 70.1, 67.6, 61.7, 57.5, 20.8, 20.7 ppm. HRMS: Calcd for C₁₉H₂₁O₉NNaS₂ [M+Na]+: 494.0550; found 494.0546 (-0.7 ppm).

### NrF2 Activation

AREc32 cells (kindly shared by Prof. C.R. Wolf) were grown in Dulbecco's modified Eagle medium (DMEM) with glutamax, supplemented with 10% fetal bovine serum (FBS), 1% penicillin-streptomycin and geneticin (0.8 mg/mL, G418) (GIBCO, Madrid, Spain) at 37 °C in a 5% CO2 atmosphere and subcultured in a new flask when confluence increased to 80%. Cells were seeded in 96-well white plates (20,000 cells/well) using 100 µL/well of culture media. After 24 h in culture, cells were treated with compounds at desired concentrations (1, 5, 10, 15 µM) for 24 h, including tert-butyl hydroquinone (TBHQ, 10 µM) as a positive control. Thereafter, luciferase activity was assessed by a bioluminescence assay using the "Luciferase assay system" (Promega E1500), following provider protocol, measuring luminescence in an ClarioStar multiwell plate reader (BMG Labtech, Germany). Treatments were performed in duplicate, and values were normalized to basal luminescence considered as 1. Nrf2 induction capacity was expressed as CD values, (concentrations required to double luciferase activity) obtained from dose-response curves generated by plotting folds induction at each concentration Vs. Log[compound] fitted by non-linear regression and data interpolated to value 2.

### MTT based cell viability evaluation

After treatments, primary mixed glial cultures and AREc32 cells were incubated for 120 minutes with tetrazolium salt (3-(4,5-dimethylthiazol-2-yl)-2,5-diphenyltetrazolium bromide solution (MTT, 0.5 mg/mL) for cell viability measurement. In this assay, MTT (yellow salt) is reduced to purple insoluble formazan crystals by oxidoreductase enzymes from viable cells. Then, the formazan crystals were solubilized by adding dimethyl sulfoxide (DMSO) to the corresponding p96-well plates. Finally, absorbance was measured at 535 nm in a microplate reader SPECTROstar Nano (BMG Labtech). Basal absorbance was set to 100 % and results were normalized to basal conditions.

### Statistical Analysis

Data are represented as mean ± S.E.M. CD values were calculated by non-linear regression analysis of individual dose-response curves. Experimental and control groups were compared using the t-test and multiple groups were compared using a one-way analysis of variance test (one-way ANOVA) followed by a Newman-Keuls post hoc test. Statistical significance was set at * p < 0.033, ** p < 0.002, and *** p < 0.001. Data was analyzed using GraphPad Prism 8.0 software.

### Cytotoxic activity

### Chemicals and Cell Lines and healthy donor primary cells

Resazurin was obtained from Sigma. Gemcitabine was purchased from Pfizer.

HaCaT cells (human keratinocytes, reference control), A549 (human lung adenocarcinoma cells), MeWo (human melanoma cells) and T24 (human bladder cancer cells) were purchased from Cell Lines Service (CLS). Cells were grown in Dulbecco's Modified Eagle's Medium (DMEM) supplemented with 100 U/mL penicillin, 100 µg/mL streptomycin and 10% fetal bovine serum. HL60 cell line (acute myeloid leukaemia, AML) was obtained from ATCC. It was cultured in IMDM medium supplemented with 100 U/mL penicillin, 100 µg/mL streptomycin and 10% fetal bovine serum. U937 (AML) and Jurkat cells (ALL) were obtained from DMSZ, Braunschweig, Germany, and ATCC, respectively. They were cultivated in RPMI medium, supplemented with 100 U/mL penicillin, 100 µg/mL streptomycin and 10% fetal bovine serum. OPM-2 cell line (MM), was obtained from DMSZ, Braunschweig, Germany and cultured in RPMI medium supplemented with 100 U/mL penicillin, 100 µg/mL streptomycin, 10% fetal bovine serum and 1X GlutaMAXTM. All cell lines were cultured at 37 ° C with 5% CO2 in a humidified incubator. Cell culture reagents were purchased from Biowest.

### Cell viability assay

Exponentially growing cells (3000-6000 cells per well) were seeded in 96-well plates. After 24 hours, cells were treated with the drugs for 72 hours. The cell viability was then estimated with the resazurin assay, a colorimetric technique based on the capability of viable cells to reduce the blue reagent resazurin to the pink-soluble product resorufin. The amount of resorufin produced is proportional to the number of viable cells. Briefly, cells were washed once with phosphate buffered saline (PBS) and 150 µL resazurin (20 µg/mL in medium) was added to each well. The plates were incubated for 3 hours at 37 °C, 5% CO₂, and finally optical densities were measured at 540 and 620 nm with a multiwell plate spectrophotometer reader (Multiskan EX Labsystems). Cell viability was calculated as a percentage compared to non-treated cells. The results were expressed as the means ± standard error of the mean (SEM). All data are from at least three independent experiments.

For statistical analysis, Student's t-test (paired, two tailed) was carried out to compare the cytotoxicity of a particular concentration of the compound between HaCaT and A549 (asterisks), or HaCaT and MeWo (cross) or HaCaT and T24 (hashes). A p value >0.05 is not considered statistically significant and is not represented by any symbol. A p value <0.05 is considered statistically significant and is indicated with * or + or #; a p value <0.01 is indicated with ** or ++ or ##; and a p value <0.001 is indicated with *** or +++ or ###.

The selectivity index (S.I.) values were calculated over each cancer cell line as the mean of the IC50 value in the non-malignant cell line (HaCaT) by the IC50 in the respective cancer cell line obtained in each independent experiment.

For the leukemic cell lines, the CCK-8 Cell Counting Kit (Dojindo Molecular Technologies) was used according to manufacturers instructions. Briefly, 50.000 cells were seeded in each well of a 96 well plate, and they were treated with increased amounts (0 - 100 µM) of each of the compounds, and incubated for 18 h at 37ºC, 5% CO2. After this period, 7 µl of the CCK-8 reagent were added to each well, and cells were further incubated for 2 h at 37ºC, 5% CO2. OD at 450 nm was obtained using a MultiskanTM GO microplate reader (Thermo Fisher Scientific, Waltham, MA, EE.UU). Cell viability in each condition was normalized to the measurement obtained in the non treated samples (100%), and were represented as average ± standard error of the mean (SEM).

To confirm the results, cell death was measured by flow cytometry using Anexin V and 7AAD Staining. 100.000 cells were seeded in each well of a 96 well plate, and they were treated with increased amounts (0 - 100 µM) of each the compounds, and incubated for 18 h at 37ºC, 5% CO2. Cells were then collected and stained using the BD Pharmingen PE-Anexin V Apoptosis Detection Kit I (BD Biosciences) according to manufacturer's instructions. Cells were acquired in a FACS Canto II cytometer (BD Biosciences) and the results were analysed using the FlowJo vX software. Cell viability was calculated as the percentage of Anexin V- 7AAD, and was normalized to the non treated controls (100%). Data were represented as average ± standard error of the mean (SEM).

## Claims

1. A compound of formula I: 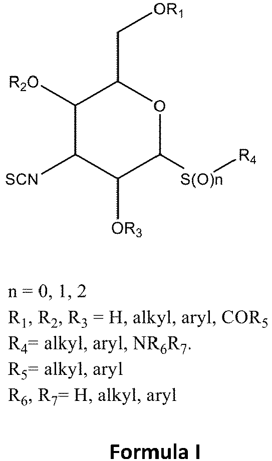 or a pharmaceutically acceptable salt thereof.

2. The compound of formula I, or a pharmaceutically acceptable salt thereof, according to claim 1, wherein
a. R₁ to R₃ are independently selected from a C₁-C₄ alkyl (preferably a C₁-C₂ alkyl), an aryl such as a phenyl group, or an acetyl group (-COCH₃); and
b. R₄ is selected from a C₁-C₄ alkyl (preferably a C₁-C₂ alkyl), or an aryl such as a substituted or unsubstituted phenyl group.

3. The compound of formula I, or a pharmaceutically acceptable salt thereof, according to claim 2, wherein:
a. R₁ to R₃ are acetyl groups (-COCH₃); and
b. R₄ is selected from a C₁-C₄ alkyl (preferably a C₁-C₂ alkyl), or an aryl such as a substituted or unsubstituted phenyl group.

4. The compound of formula I, or a pharmaceutically acceptable salt thereof, according to claim 3, wherein the compound is of formula II: 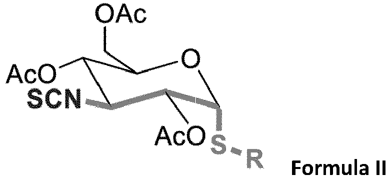 and wherein R is selected from a methyl group (compound **13α**), an ethyl group (compound **14α**), or a phenyl group (compound **15α**).

5. The compound of formula I, or a pharmaceutically acceptable salt thereof, according to claim 3, wherein the compound is of formula III: 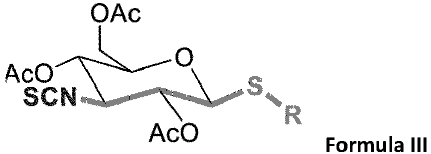 and R is selected from a methyl group (compound **13β**), an ethyl group (compound **14β**), or a phenyl group (compound **15β**).

6. The compound of formula I, or a pharmaceutically acceptable salt thereof, according to claim 3, wherein the compound is of formula IV: 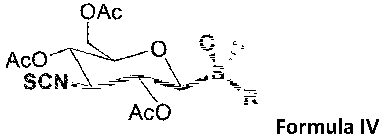 and R is selected from a methyl group (compound (*Sₛ*)-**16β**), an ethyl group (compound (*Sₛ*)-**17β**), or a phenyl group (compound (*Sₛ*)-**18β**).

7. The compound of formula I, or a pharmaceutically acceptable salt thereof, according to claim 3, wherein the compound is of formula V: 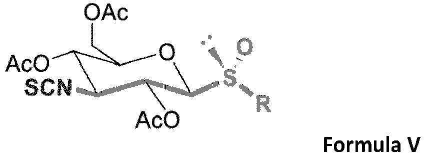 and R is selected from an ethyl group (compound (*Rₛ*)-**17β**), or a phenyl group (compound (*Rₛ*)-**18β**).

8. The compound of formula I, or a pharmaceutically acceptable salt thereof, according to claim 3, wherein the compound is of formula VI: 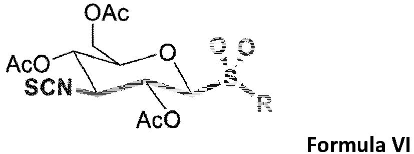 and R is selected from a methyl group (compound **19β**), an ethyl group (compound **20β**), or a phenyl group (compound 21β).

9. The compound of formula I, or a pharmaceutically acceptable salt thereof, according to claim 3, wherein the compound is of formula VII: 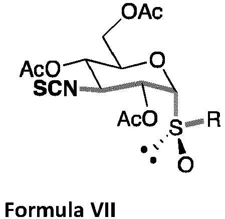 and R is selected from an ethyl group (compound (*R*ₛ)-**17α**), or a phenyl group (compound (*R*ₛ)-**18α**).

10. The compound of formula I, or a pharmaceutically acceptable salt thereof, according to claim 3, wherein the compound is of formula VIII: 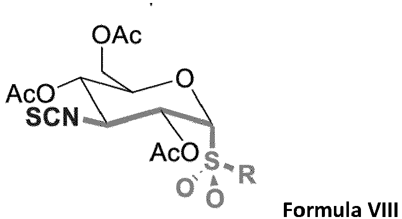 and R can be selected from an ethyl group (compound **20α**), or a phenyl group (compound **21α**).

11. A pharmaceutical composition comprising a compound of formula I as defined in any of claims 1 to 10.

12. The compound of any of claims 1 to 10 or the pharmaceutical composition according to claim 11, for use in a method of treatment of cancer.

13. The compound or composition for use according to claim 12, wherein the cancer is a solid tumour or a haematological cancer.

14. The compound or composition for use according to claim 13, wherein the cancer is a solid tumor selected from the list consisting of lung cancer, in particular lung adenocarcinoma, melanoma, or bladder cancer.

15. The compound or composition for use according to claim 13, wherein the cancer is a haematological cancer selected from the list consisting of acute myeloid leukaemia, multiple myeloma and acute lymphoblastic leukaemia
